(19) Europäisches Patentamt
European Patent Office
Office européen des brevets

(11) Veröffentlichungsnummer: **0 387 613 A1**

# (12) EUROPÄISCHE PATENTANMELDUNG

(21) Anmeldenummer: **90103948.7**

(22) Anmeldetag: **01.03.90**

(51) Int. Cl.[5]: **C07D 495/14, A61K 31/55, //(C07D495/14,333:00,249:00, 243:00)**

(30) Priorität: **03.03.89 DE 3906875**

(43) Veröffentlichungstag der Anmeldung: **19.09.90 Patentblatt 90/38**

(84) Benannte Vertragsstaaten: **AT BE CH DE DK ES FR GB GR IT LI LU NL SE**

(71) Anmelder: **BOEHRINGER INGELHEIM KG**

**D-6507 Ingelheim am Rhein(DE)**

(84) **BE CH DE DK ES FR GR IT LI LU NL SE AT**

Anmelder: **BOEHRINGER INGELHEIM INTERNATIONAL G.M.B.H.**

**D-6507 Ingelheim am Rhein(DE)**

(84) **GB**

(72) Erfinder: **Stransky, Werner, Dr.**
**Im Hippel 24**
**D-6535 Gau-Algesheim(DE)**
Erfinder: **Weber, Karl-Heinz, Dr.**
**Kaiser-Karl-Strasse 11**
**D-6535 Gau-Algesheim(DE)**
Erfinder: **Walther, Gerhard, Dr.**
**Pfarrer-Heberer-Strasse 37**
**D-6530 Bingen(DE)**
Erfinder: **Küfner-Mühl, Ulrike, Dr.**
**Am Rübenacker 5**
**D-6500 Mainz(DE)**
Erfinder: **Birke, Franz, Dr.**
**Albrecht-Dürer-Strasse 23**
**D-6507 Ingelheim am Rhein(DE)**
Erfinder: **Bechtel, Wolf-Dietrich, Dr.**
**Mühlstrasse 3**
**D-6531 Appenheim(DE)**
Erfinder: **Muacevic, Gojko, Dr.**
**In der Dörrwiese 13**
**D-6507 Ingelheim am Rhein(DE)**
Erfinder: **Heuer, Hubert, Dr.**
**Am Alten Weg 29**
**D-6500 Mainz 32(DE)**

## (54) Neue Thienodiazepine.

(57) Die Anmeldung betrifft Thienodiazepine der allgemeinen Formel Ia und Ib, Verfahren zu ihrer Herstellung und ihre Verwendung als Arzneimittel.

EP 0 387 613 A1

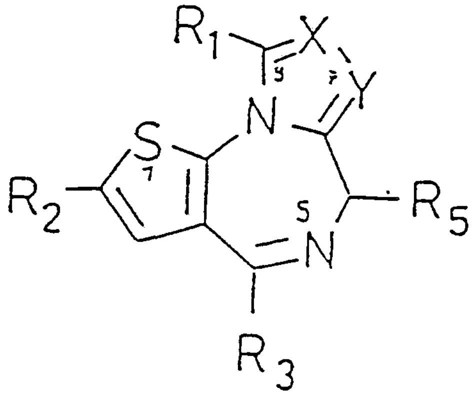

Ia

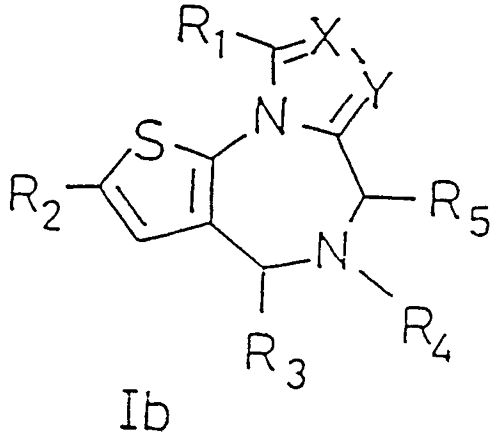

Ib

## Neue Thienodiazepine

Die Erfindung betrifft neue Thienodiazepine, Verfahren zu ihrer Herstellung und ihre Verwendung als Arzneimittel.

Die neuen Verbindungen entsprechen der allgemeinen Formel

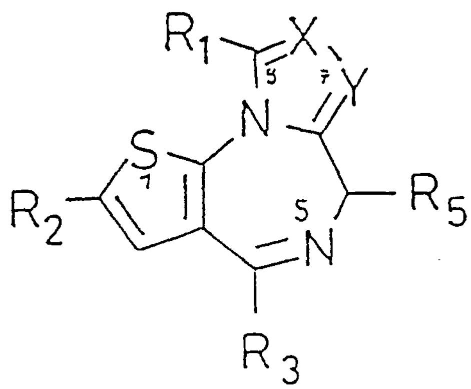

Ia

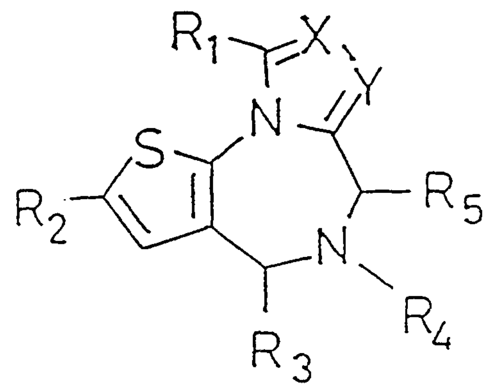

Ib

worin

$R_1$ Wasserstoff, eine verzweigte oder unverzweigte Alkylgruppe mit 1 bis 4 Kohlenstoffatomen, bevorzugt Methyl, die gegebenenfalls durch Hydroxy oder Halogen substituiert sein kann, eine Cyclopropylgruppe, eine Cyclobutyl, eine verzweigte oder unverzweigte Alkoxygruppe mit 1 bis 4 Kohlenstoffatomen, bevorzugt Methoxy, Halogen, bevorzugt Chlor oder Brom;

$R_2$

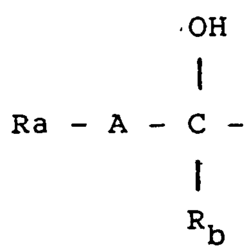

oder

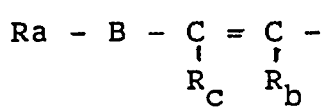

oder

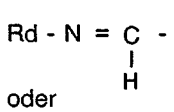

oder

Re - d - $CH_2$-

worin

A $-(CH_2)_n-$ oder eine verzweigte Alkylgruppe mit n Kohlenstoffatomen

B $-(CH_2)_m-$ oder eine verzweigte Alkylgruppe mit m Kohlenstoffatomen

D Sauerstoff, Schwefel oder NH bedeutet,

worin

Ra Wasserstoff, verzweigtes oder unverzweigtes Alkyl mit 1 bis 4 Kohlenstoffatomen, gegebenenfalls durch Halogen substituiert, $C_1$ bis $C_8$ Alkoxy, bevorzugt $C_1$ bis $C_4$-Alkoxy, eine gegebenenfalls substituierte

Cycloalkylgruppe mit 3 bis 6 Kohlenstoffatomen, gegebenenfalls substituiertes Aryl-, Aryloxy-, Aralkyl-, Aralkyloxy, gegebenenfalls substituiertes Heteroaryl, ein gegebenenfalls substituierter gesättigter oder ungesättigter 5-, 6- oder 7- gliedriger heterocyclischer Ring, einen Rest der Formel

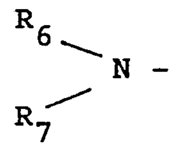

wobei

$R_6$ Wasserstoff, eine verzweigte oder unverzweigte Alkyl-, Alkenyl- oder Alkinylgruppe mit 1 - 10 Kohlenstoffatomen, bevorzugt 1 - 4, Kohlenstoffatomen, die gegebenenfalls durch Halogen, Carboxy, Alkoxycarbonyl mit 1 bis 6, bevorzugt 1 bis 4, Kohlenstoffatomen, Phenyl, substituiertes Phenyl, oder durch einen C- oder N-verknüpften Heterocyclus substituiert sein kann, wobei die Kohlenstoffkette durch Stickstoff, Sauerstoff oder Schwefel unterbrochen sein kann,

eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,

$R_6$ eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen,

$R_6$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6-oder 7-gliedriger heterocyclischer Ring, Phenyl, substituiertes Phenyl;

$R_7$ Wasserstoff, eine verzweigte oder unverzweigte Alkyl-, Alkenyl- oder Alkinylgruppe mit 1 - 10 Kohlenstoffatomen, bevorzugt 1-4 Kohlenstoffatomen, die gegebenenfalls durch Halogen, Carboxy, Alkoxycarbonyl, Phenyl, substituiertes Phenyl, oder durch einen C- oder N-verknüpften Heterocyclus substituiert sein kann, wobei die Kohlenstoffkette durch Stickstoff, Sauerstoff oder Schwefel unterbrochen sein kann,

eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,

$R_7$ eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen, oder

$R_6$ und $R_7$ bilden zusammen mit dem Stickstoffatom einen gesättigten oder ungesättigten gegebenenfalls ein- oder mehrfach

durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;

$R_7$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6-, oder 7-gliedriger heterocyclischer Ring, Phenyl, substituiertes Phenyl;

$R_a$ ein gegebenenfalls substituierter Imido- oder Phthalimidorest;

$R_b$ entsprechend einem Rest wie unter $R_a$ definiert, bevorzugt Wasserstoff oder verzweigtes oder unverzweigtes Alkyl mit 1 bis 4 Kohlenstoffatomen, Phenyl, substituiertes Phenyl;

$R_c$ Wasserstoff, Aryl, bevorzugt Phenyl, substituiertes Phenyl, $C_1$ - $C_4$-Alkyl,

$R_d$ eine verzweigte oder unverzweigte Alkyl-, Alkenyl- oder Alkinylgruppe mit 1 bis 10 Kohlenstoffatomen, bevorzugt mit 5 bis 10 Kohlenstoffatomen, im Substitutionsfall bevorzugt mit 1 bis 4 Kohlenstoffatomen, die gegebenenfalls durch Carboxy, Alkoxycarbonyl mit 1 bis 6 , bevorzugt 1 bis 4 Kohlenstoffatomen, Phenyl, substituiertes Phenyl, oder durch einen C- oder N-verknüpften 5-, 6-, 7-gliedrigen Heterocyclus substituiert sein kann,

$R_d$ ein gegebenenfalls ein- oder mehrfach durch verzweigtes oder unverzweigtes Alkyl mit 1 bis 4 Kohlenstofftomen substituierter, gesättigter oder ungesättigter über ein Kohlenstoffatom oder Stickstoffatom

verknüpfter 5-, 6- oder 7-gliedriger heterocyclischer Ring, Phenyl, substituiertes Phenyl;
$R_e$ für dun Fall, daß D die Bedeutung NH aufweist bedeutet, $R_e = R_d$,
für den Fall, daß D die Bedeutung Sauerstoff oder Schwefel aufweist, bedeutet,
$R_e$ $C_1$ - $C_8$, bevorzugt $C_1$ - $C_4$ - Alkyl, gegebenenfalls substituiert durch Phenyl oder substituiertes Phenyl, Phenyl und substituiertes Phenyl;
n eine der Zahlen 0, 1, 2, 3, 4, 5, 6, 7 oder 8, bevorzugt 0, 1 oder 2;
m eine der Zahlen 0, 1, 2, 3, 4, 5, 6 oder 7 bevorzugt 0, 1 oder 2;
$R_2$ ein Rest der allgemeinen Formel
$R_f$-O-$SO_2$-
worin
$R_f$ verzweigtes oder unverzweigtes Alkyl mit 1 bis 10, bevorzugt 1 bis 4 Kohlenstoffatomen, das gegebenenfalls durch Halogen, Phenyl, substituiertes Phenyl substituiert sein kann;
eine Cycloalkylgruppe mit 1 bis 6 Kohlenstoffatomen, gegebenenfalls substituiert durch Halogen, Phenyl oder substituiertes Phenyl,
$R_f$ Phenyl, substituiertes Phenyl, Heteroaryl, substituiertes Heteroaryl;
$R_2$ einen Rest der allgemeinen Formel

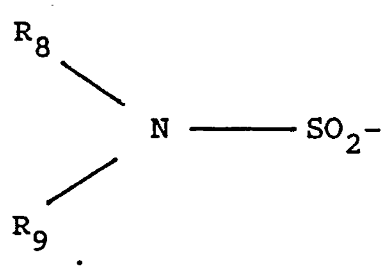

worin
$R_8$ Wasserstoff, eine verzweigte oder unverzweigte gegebenenfalls durch Stickstoff, Sauerstoff oder Schwefel unterbrochene Alkyl-, Alkenyl oder Alkinylgruppe mit 1 - 10 Kohlenstoffatomen, bevorzugt 1 -4 Kohlenstoffatomen, die gegebenenfalls durch{Halogen, Hydroxy, Phenyl, substituiertes Phenyl, Alkyloxycarbonyl mit 1 bis 8 - bevorzugt 1-4 -Kohlenstoffatomen in der Alkylkette, Carboxy, eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,
eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen, oder durch einen C- oder N-verknüpften Heterocyclus} substituiert sein kann;
$R_8$ Phenyl, substituiertes Phenyl
$R_8$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6-oder 7-gliedriger heterocyclischer Ring,
$R_9$ Wasserstoff, eine verzweigte oder unverzweigte gegebenenfalls durch Stickstoff, Sauerstoff oder Schwefel unterbrochene Alkyl-, Alkenyl- oder Alkinylgruppe mit 1 - 10 Kohlenstoffatomen, bevorzugt 1 -4 Kohlenstoffatomen, die gegebenenfalls durch{Halogen, Hydroxy, Carboxy, Alkoxycarbonyl, Phenyl, substituiertes Phenyl, eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,
eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen, oder durch einen C- oder N-verknüpften Heterocyclus} substituiert sein kann,
$R_9$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6-, oder 7-gliedriger heterocyclischer Ring, Phenyl, substituiertes Phenyl; oder
$R_8$ und $R_9$ bilden zusammen mit dem Stickstoffatom einen gesättigten oder ungesättigten gegebenenfalls ein- oder mehrfach
durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;
$R_a$ einen Rest der allgemeinen Formel

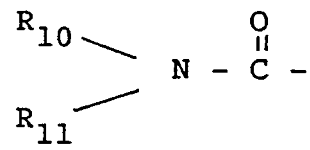

worin
$R_{10}$ und $R_{11}$, die gleich oder verschieden sein können, Wasserstoff, Phenyl, substituiertes Phenyl, eine gegebenenfalls substituierte Cycloalkylgruppe mit 3 bis 6 Kohlenstoffatomen, eine verzweigte oder unverzweigte Alkyl-, Alkenyl-oder Alkinylgruppe mit 1 bis 10 - bevorzugt 1 - 4 - Kohlenstoffatomen, die gegebenenfalls durch {Halogen, Hydroxy, Nitro, Phenyl, substituiertes Phenyl, Amino, substituiertes Amino, Alkoxy 1 - 8, bevorzugt 1 - 4 oder im Fall von $R_{10}$ = Wasserstoff und Alkyl durch eine Esterfunktion oder ein Säureamid der allgemeinen Formel

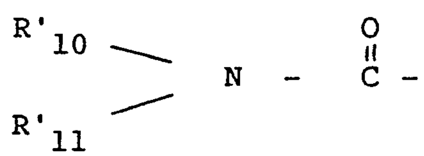

worin
$R'_{10}$ und $R'_{11}$ dieselbe Bedeutung wie $R_{10}$ und $R_{11}$, haben kann, Phenyl, substituiertes Phenyl} substituiert sein kann;
$R_{10}$ oder $R_{11}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoff gebundener 5-, 6- oder 7-gliedriger heterocyclischer Ring;
oder
$R_{10}$ und $R_{11}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann.
$R_2$ -CHO oder COOH;
$R_3$ Phenyl, wobei der Phenylring ein oder mehrfach, bevorzugt in 2-Stellung, durch Methyl, bevorzugt Halogen, bevorzugt Brom, besonders bevorzugt Chlor, Nitro und/oder Trifluormethyl substituiert sein kann;
$R_3$ einen Rest der allgemeinen Formel

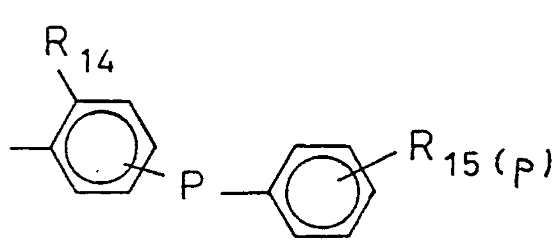

worin
$R_{14}$ Wasserstoff oder Chlor
P den Rest - $(CH_2)_m$ mit m = 0, 1, 2, 3 oder 4, $-OCH_2-$, $-CH_2OCH_2-$
P 0, 1, 2, oder 3
$R_{15}$ Wasserstoff, $CF_3$, Chlor, Fluor, $C_1$ - $C_4$-Alkyl, $C_1$ - $C_4$ - Alkoxy, oder den Rest

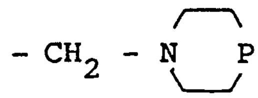

worin P, Sauerstoff, Schwefel oder N-$CH_3$ bedeutet,
mit der Maßgabe, daß wenn $R_{14}$ Wasserstoff bedeutet, $R_{15}$, in der 3 oder 4 Position ist, wenn $R_{14}$ Chlor bedeutet, $R_{15}$ in der 4 Position ist, wenn $R_{15}$ Chlor, Fluor, oder Methyl bedeutet, ist p 3, wenn $R_{15}$ tert.-Butyl oder

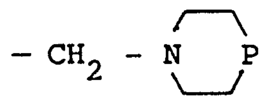

bedeutet ist p = 1; wenn p = 2 ist, ist $R_{15}$ nicht gleichzeitig in der 2- oder 6-Position;
$R_3$ Pyridyl, substituiertes Pyridyl, Thienyl oder substituiertes Thienyl, wobei der Heterocyclus bevorzugt über die 2-Stellung verknüpft ist;
$R_4$ Wasserstoff, verzweigtes oder unverzweigtes Alkyl mit 1 bis 4 Kohlenstoffatomem, Formyl, verzweigter oder unverzweigter Acylrest mit 1 bis 4 Kohlenstoffatomen;
$R_5$ Wasserstoff, Hydroxy, verzweigtes oder unverzweigtes Alkyl mit 1 bis 6, bevorzugt 1 bis 4 Kohlenstoffatomen, besonders bevorzugt Methyl, gegebenenfalls ist die Alkylgruppe durch Hydroxy, Alkylsulfonyloxy, bevorzugt Methylsulfonyloxy, -$NR_8R_9$ oder -CO-$NR_{12}R_{13}$ substituiert,
wobei $R_8$ und $R_9$ die zuvor genannte Bedeutung aufweisen und
$R_{12}$ Wasserstoff, eine verzweigte oder unverzweigte Alkyl-, Alkenyl- oder Alkinylgruppe mit 1 bis 18 Kohlenstoffatomen, bevorzugt mit 1 bis 6, besonders bevorzugt 1 bis 4, die gegebenenfalls durch{Halogen, Hydroxy, Nitro, Amino, substituiertes Amino, Alkoxy, bevorzugt Methoxy oder im Fall von $R_{13}$ = Wasserstoff oder Alkyl, durch eine Esterfunktion oder durch ein Säureamid der allgemeinen Formel

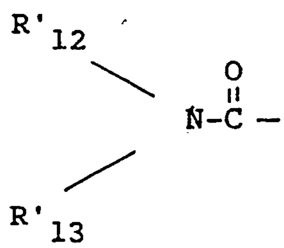

worin $R'_{12}$ und $R'_{13}$, dieselbe Bedeutung wie $R_{12}$ und $R_{13}$ haben können, Phenyl, substituiertes Phenyl} substituiert sein kann;
$R_{13}$ Wasserstoff, eine verzweigte oder unverzweigte Alkyl-, Alkenyl- oder Alkinylgruppe mit 1 bis 18 Kohlenstoffatomen, bevorzugt mit 1 bis 6, besonders bevorzugt 1 bis 4, die gegebenenfalls durch Halogen, Hydroxy, Nitro, Amino, substituiertes Amino, Alkoxy, bevorzugt Methoxy; Phenyl substituiert sein kann;
ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6- oder 7-gliedriger heterocyclischer Ring;
oder
$R_{12}$ und $R_{13}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann,
$R_5$ Alkylcarbonyloxy mit 1 bis 4 Kohlenstoffatomen in der verzweigten oder unverzweigten Alkylkette, Alkoxycarbonyl mit 1 bis 4 Kohlenstoffatomen in der verzweigten oder unverzweigten Alkylkette, Carboxyalkyl mit 1 bis 4 Kohlenstoffatomen in der verzweigten oder unverzweigten Alkylkette, Alkoxycarbonylalkyl mit 1 bis 4 Kohlenstoffatomen in den verzweigten oder unverzweigten Alkylresten;
X/Y unabhängig voneinander C-$R_1$ oder N, aber nicht beide C-$R_1$, mit $R_1$ bevorzugt Wasserstoff oder Methyl,
oder Y die Gruppe C-COOR', wobei R' Alkyl oder Wasserstoff bedeutet und X Stickstoff bedeuten können;

gegebenenfalls in Form ihrer Racemate, ihrer Enantiomere, ihrer Diastereomere und ihrer Gemische sowie gegebenenfalls ihre physiologisch unbedenklichen Säureadditionssalze.

Bevorzugt sind Verbindungen der allgemeinen Formel Ia

worin

$R_1$ eine verzweigte oder unverzweigte Alkylgruppe mit 1 bis 3 Kohlenstoffatomen, bevorzugt Methyl und Ethyl die gegebenenfalls durch Hydroxy oder Halogen substituiert sein können, eine Cyclopropylgruppe, eine verzweigte oder unverzweigte Alkoxygruppe mit 1 bis 3 Kohlenstoffatomen, bevorzugt Methoxy;

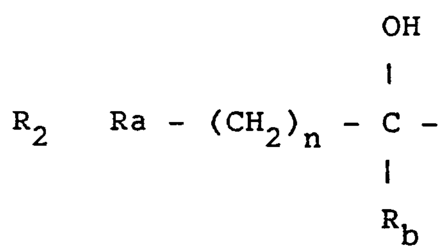 oder

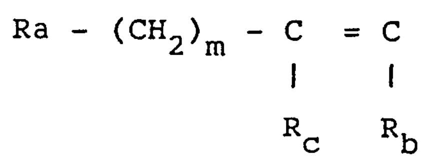 oder

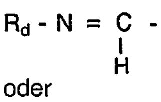

oder

$R_e - D - CH_2-$,

worin D Sauerstoff, Schwefel oder NH bedeutet,

worin

Ra Wasserstoff, verzweigtes oder unverzweigtes Alkyl mit 1 bis 4 Kohlenstoffatomen gegebenenfalls durch Halogen substituiert, gegebenenfalls substituiertes Aryl, bevorzugt substituiertes Phenyl, wobei der Substituent auch einen Rest der allgemeinen Formel

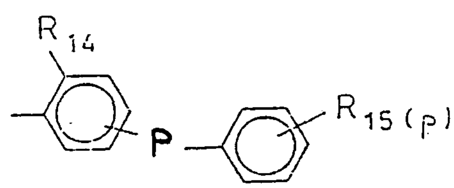

worin P, $R_{15}$ und p wie zuvor definiert sein können, gegebenenfalls substituiertes Heteroaryl, einen Rest der Formel

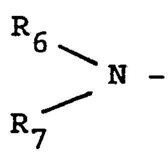

wobei

$R_6$ Wasserstoff, eine verzweigte oder unverzweigte Alkyl-, oder Alkenylgruppe mit 1 - 10 Kohlenstoffatomen, bevorzugt 1 -4 Kohlenstoffatomen, die gegebenenfalls durch Halogen, Phenyl, substituiertes Phenyl, oder durch einen C- oder N-verknüpften Heterocyclus substituiert sein kann, wobei die Kohlenstoffkette

durch Stickstoff, Sauerstoff oder Schwefel unterbrochen sein kann,
eine verzweigte oder unverzweigte Alkylcarbonylgruppe mit 1-4 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 4 Kohlenstoffatomen substituierte Aminogruppe, wobei die Alkylgruppe durch Halogen oder Hydroxy substituiert sein kann,
$R_6$ eine gegebenenfalls substituierte Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen,
$R_6$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6-oder 7-gliedriger heterocyclischer Ring, Phenyl- gegebenenfalls substituiert;
$R_7$ Wasserstoff, eine verzweigte oder unverzweigte Alkyl-, Alkenyl- oder Alkinylgruppe mit 1 - 4 Kohlenstoffatomen
oder
$R_6$ und $R_7$ bilden zusammen mit dem Stickstoffatom einen gesättigten oder ungesättigten gegebenenfalls ein- oder mehrfach
durch verzweigte oder unverzweigte Alkylgruppen mit 1 bis 4 Kohlenstoffatomen substituierten 5-, oder 6-Ring der als weitere Heteroatome Stickstoff oder Sauerstoff enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;
$R_a$ ein gegebenenfalls substituierter Phthalimidorest;
$R_b$ Wasserstoff oder verzweigtes oder unverzweigtes Alkyl mit 1 bis 4 Kohlenstoffatomen, Phenyl, substituiertes Phenyl;
$R_c$ Wasserstoff, Phenyl oder substituiertes Phenyl;
$R_d$ $C_5$ bis $C_{10}$-Alkyl, gegebenenfalls substituiertes $C_1$ bis $C_4$-Alkyl, Phenyl, substituiertes Phenyl;
$R_e$ für den Fall, daß D die Bedeutung NH aufweist, bedeutet $R_e = R_d$, für den Fall, daß D die Bedeutung Sauerstoff oder Schwefel aufweist, bedeutet,
$R_e$ $C_1$ - $C_4$-Alkyl-Phenyl, Phenyl, substituiertes Phenyl;
n eine der Zahlen 0, 1, 2, 3, 4, 5, 6, 7 oder 8, bevorzugt 0, 1 oder 2;
m eine der Zahlen 0, 1, 2, 3, 4, 5, 6 oder 7 bevorzugt 0, 1 oder 2;
$R_2$ ein Rest der allgemeinen Formel
$R_f$-O-$SO_2$-
worin
$R_f$ verzweigtes oder unverzweigtes Alkyl mit 1 bis 4 Kohlenstoffatomen, das gegebenenfalls durch Halogen, Phenyl, substituiertes Phenyl substituiert sein kann;
$R_f$ Phenyl, substituiertes Phenyl, Heteroaryl, substituiertes Heteroaryl;
$R_2$ einen Rest der allgemeinen Formel

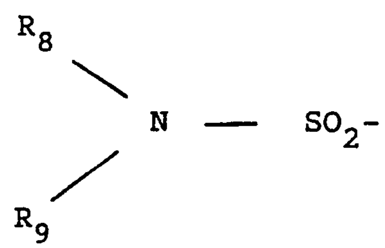

worin
$R_8$ Wasserstoff, eine verzweigte oder unverzweigte Alkyl- oder Alkenylgruppe mit 1 - 10 Kohlenstoffatomen, bevorzugt 1 -4 Kohlenstoffatomen, wobei die Kohlenstoffkette durch Stickstoff, Sauerstoff oder Schwefel unterbrochen sein kann, die gegebenenfalls durch {Halogen, Hydroxy, Phenyl, substituiertes Phenyl, eine verzweigte oder unverzweigte Alkylcarbonylgruppe mit 1-4 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen ,substituierte Aminogruppe, wobei die Alkylgruppe durch Halogen oder Hydroxy substituiert sein kann,
ein gegebenenfalls substituiertes Phenylcarbonyl, gegebenenfalls substituiertes Phenylsulfonyl oder Tolylsulfonyl, eine Alkylsulfonylgruppe mit 1 bis 4 Kohlenstoffatomen,oder durch einen C-verknüpften Heterocyclus} substituiert sein kann,
$R_8$ Phenyl, substituiertes Phenyl;

$R_8$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom verknüpfter 5-, 6- oder 7-gliedriger heterocyclischer Ring;

$R_9$ Wasserstoff, eine verzweigte oder unverzweigte Alkylgruppe mit 1 - 4 Kohlenstoffatomen oder

$R_8$ und $R_9$ bilden zusammen mit dem Stickstoffatom einen gesättigten oder ungesättigten gegebenenfalls ein- oder mehrfach

durch verzweigte oder unverzweigte Alkylgruppen mit 1 bis 4 Kohlenstoffatomen substituierten 5-, 6-Ring, der als weitere Heteroatome Stickstoff oder Sauerstoff enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;

$R_9$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom verknüpfter 5- 6-, oder 7-gliedriger heterocyclischer Ring;

$R_2$ -CHO oder COOH;

$R_3$ Phenyl, wobei der Phenylring in 2-Stellung durch Halogen, bevorzugt Brom, besonders bevorzugt Chlor substituiert sein kann, 2-Thienyl;

$R_3$ einen Rest der allgemeinen Formel

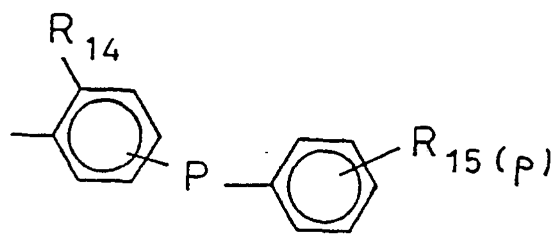

P = wie zuvor definiert,

p = 1, 2 oder 3

$R_{15}$ = $OCH_3$, $OC_2C_5$,

$R_5$ Wasserstoff, Hydroxy, verzweigtes oder unverzweigtes Alkyl mit 1 bis 6, bevorzugt 1 bis 4 Kohlenstoffatomen, besonders bevorzugt Methyl,

X/Y unabhängig voneinander C-$R_1$ oder N, aber nicht beide C-$R_1$, mit $R_1$ bevorzugt Wasserstoff oder Methyl, bevorzugt ist Y = Stickstoff.

Besonders bevorzugt sind Verbindungen der allgemeinen Formel Ia, worin

$R_1$ eine Methylgruppe, eine Ethylgruppe die gegebenenfalls durch Hydroxy oder Halogen substituiert sein können, eine Cyclopropylgruppe, eine Methoxygruppe;

$$R_2 \quad Ra - (CH_2)_n - \overset{\overset{OH}{|}}{\underset{\underset{R_b}{|}}{C}} - \qquad \text{oder}$$

$$Ra - (CH_2)_m - \underset{\underset{R_c}{|}}{C} = \underset{\underset{R_b}{|}}{C} \qquad \text{oder}$$

Re - D - $CH_2$ -

worin D wie zuvor definiert ist,

oder

$R_d$ - N = CH -

Ra Wasserstoff, verzweigtes oder unverzweigtes Alkyl mit 1 bis 4 Kohlenstoffatomen gegebenenfalls durch Halogen substituiert, Phenyl, gegebenenfalls ein-oder mehrfach substituiert durch Halogen, Amino, Alkylamino, Dialkylamino, $C_1$-$C_4$ Alkyl $C_1$-$C_4$-Halogenalkyl, $C_1$-$C_4$ Alkoxy und/oder $C_1$-$C_4$-Alkoxycarbonyl, gegebenenfalls substituiertes Benzodioxolan, gegebenenfalls substituiertes Furan, Thiophen oder Pyridin, einen Rest der Formel

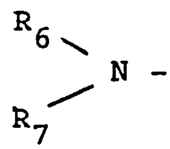

wobei

$R_6$ Wasserstoff, eine verzweigte oder unverzweigte Alkylgruppe mit 1 -4 Kohlenstoffatomen, die gegebenenfalls durch Halogen, Phenyl, substituiertes Phenyl, substituiert sein kann, eine Allylgruppe, eine Propargylgruppe eine verzweigte oder unverzweigte Alkylcarbonylgruppe mit 1-4 Kohlenstoffatomen, wobei die Alkylgruppe durch Halogen oder Hydroxy substituiert sein kann,

$R_6$ eine gegebenenfalls substituierte Phenylcarbonyl, eine Phenylsulfonyl oder Tolylsulfonyl, eine Alkylsulfonylgruppe mit 1 bis 4 Kohlenstoffatomen,

$R_7$ Wasserstoff, eine verzweigte oder unverzweigte Alkyl- und Alkenyl mit 1 - 4 Kohlenstoffatomen;

oder

$R_6$ und $R_7$ bilden zusammen mit dem Stickstoffatom einen gesättigten gegebenenfalls ein- oder zweifach durch verzweigte oder unverzweigte Alkylgruppen mit 1 bis 3 Kohlenstoffatomen substituierten 5-, oder 6-Ring der als weitere Heteroatome Stickstoff oder Sauerstoff enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;

$R_a$ ein Phthalimidorest;

$R_b$ Wasserstoff oder verzweigtes oder unverzweigtes Alkyl mit 1 bis 4 Kohlenstoffatomen, Phenyl, gegebenenfalls substituiert durch Halogen, $C_1$-$C_4$-Alkyl $C_1$-$C_4$-Halogen-alkyl, $C_1$-$C_4$-Alkoxy, $C_1$-$C_4$ Alkoxycarbonyl, Amino, Alkylamino, Dialkylamino;

$R_c$ Wasserstoff oder Phenyl

$R_e$/$R_d$

Phenyl gegebenenfalls substituiert durch $C_1$ - $C_4$-Alkyl, $C_1$ - $C_4$-Halogenalkyl, $C_1$ - $C_4$-Alkoxy oder $C_1$ -$C_4$-Halogenalkoxy,

n eine der Zahlen 0, 1, 2, 3, 4, 5, 6, 7 oder 8, bevorzugt 0, 1 oder 2;

m eine der Zahlen 0, 1, 2, 6 oder 7;

$R_2$ ein Rest der allgemeinen Formel

$R_f$-O-$SO_2$-

worin

$R_f$ verzweigtes oder unverzweigtes Alkyl mit 1 bis 4 Kohlenstoffatomen, das gegebenenfalls durch Halogen, gegebenenfalls ein- oder mehrfach durch Halogen, Amino, Alkylamino, Dialkylamino, $C_1$-$C_4$ Alkyl $C_1$-$C_4$-Halogenalkyl, $C_1$-$C_4$- Alkoxy und/oder $C_1$-$C_4$ -Alkoxycarbonyl substituiertes Phenyl substituiert sein kann;

$R_f$ Phenyl, substituiertes Phenyl, Heteroaryl, substituiertes Heteroaryl;

$R_2$ einen Rest der allgemeinen Formel

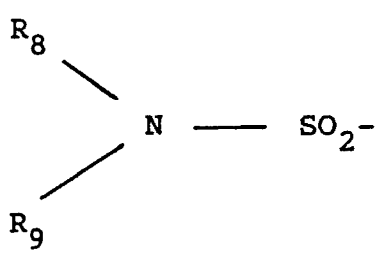

worin

$R_8$ Wasserstoff, eine verzweigte oder unverzweigte Alkylgruppe mit 1 -4 Kohlenstoffatomen, die gegebenenfalls durch Halogen, Hydroxy, Phenyl, substituiertes Phenyl, eine verzweigte oder unverzweigte Alkylcarbonylgruppe mit 1-4 Kohlenstoffatomen, ein gegebenenfalls substituiertes Phenylcarbonyl, gegebenenfalls substituiertes Phenylsulfonyl oder Tolylsulfonyl, eine Alkylsulfonylgruppe mit 1 bis 4 Kohlenstoffatomen, substituiert sein kann.

$R_9$ Wasserstoff oder

$R_8$ und $R_9$ bilden zusammen mit dem Stickstoffatom einen gesättigten gegebenenfalls ein- oder zweifach durch verzweigte oder unverzweigte Alkylgruppen mit 1 bis 4 Kohlenstoffatomen substituierten 5-oder 6-Ring, der als weiteres Heteroatom Stickstoff oder Sauerstoff enthalten kann, wobei jedes weitere Stickstoffatom gegebenenfalls durch Methyl, Ethyl oder Isopropyl substituiert sein kann;

$R_2$ -CHO oder COOH;

$R_3$ Phenyl, wobei der Phenylring in 2-Stellung durch Halogen, bevorzugt Brom, besonders bevorzugt Chlor substituiert sein kann;

$R_3$ den 4-[2-(3,4,5-Trimethoxyphenyl)-ethyl]-phenyl-Rest;

$R_5$ Wasserstoff, Hydroxy, oder Methyl;

X/Y beide Stickstoff oder X = CH und Y = Stickstoff.

Als Alkylgruppen (auch soweit sie Bestandteil anderer Reste sind) werden beispielsweise genannt: Methyl, Ethyl, Propyl, iso-Propyl, Butyl, iso-Butyl, sec.-Butyl, tert-Butyl, Pentyl, iso-Pentyl, Hexyl, Heptyl, Octyl, Nonyl und Dekanyl.

Als Alkenylgruppen werden beispielsweise oben genannte Alkylgruppen bezeichnet soweit sie mindestens eine Doppelbindung aufweisen, wie zum Beispiel Vinyl (soweit keine unbeständigen Enamine gebildet werden), Propenyl, iso-Propenyl, Butenyl, Pentenyl, Hexenyl.

Als Alkinylgruppen werden beispielsweise oben genannte Alkylgruppen bezeichnet, soweit sie mindestens eine Dreifachbindung aufweisen, wie zum Beispiel Propargyl, Butinyl, Pentinyl, Hexinyl.

Als Cycloalkylreste mit 3 - 6 Kohlenstoffatomen werden beispielsweise Cyclopropyl, Cyclobutyl, Cyclopentyl oder Cyclohexyl bezeichnet, die durch verzweigtes oder unverzweigtes Alkyl mit 1 bis 4 Kohlenstoffatomen, Hydroxy, und/oder Halogen substituiert sein können.

Als Aryl-, Aryloxy oder Aralkylreste werden aromatische Ringsysteme mit bis zu 10 Kohlenstoffatomen verstanden, wie z. B. Phenyl, Naphthyl, die durch Hydroxy, Halogen, Amino, Alkylamino, Dialkylamino, Cyano, Cycloalkyl, verzweigtes oder unverzweigtes Alkyl mit 1 bis 4 Kohlenstoffatomen, verzweigtes oder unverzweigtes Alkyloxy mit 1 bis 4 Kohlenstoffatomen, verzweigtes oder unverzweigtes Alkoxycarbonyl, substituiert sein können. Als Beispiele für Aralkyl- oder Aralkyloxyreste seien genannt, gegebenenfalls substituiertes Phenyl, das über eine $C_1$ bis $C_4$ Alkylen- oder Alkylenoxykette verknüpft ist.

Beispielhaft für substituiertes Phenyl werden genannt:

3-Chlorphenyl, 4-Chlorphenyl, 3-Bromphenyl, 4-Bromphenyl, 4-Fluormethyl, 2-Chlorphenyl, 2-Bromphenyl, 3-Fluorphenyl, 2,3-Dichlorphenyl, 2-Methylphenyl, 4-Methylphenyl, 3-Ethylphenyl, 4-Propylphenyl, 4-Isopropylphenyl, 4-Butylphenyl, 4-tert-Butylphenyl, 4-Iso-butylphenyl, 4-Pentylphenyl, 2,4-Dimethylphenyl, 2-Trifluormethylphenyl, 3-Trifluormethylphenyl, 4-Trifluormethylphenyl, 2-Methoxyphenyl, 4-Methoxyphenyl, 3-Ethoxyphenyl, 2-Propoxyphenyl, 4-Butoxyphenyl, 2,4-Dimethoxyphenyl, 3,4,5-Trimethoxyphenyl, 2-Chlorphenoxy, 2,3-Dichlorphenoxy, 2-Methylphenoxy, 4-Butylphenoxy, 2,4-Dimethylphenoxy, 2-Trifluormethylphenoxy, 2-Methoxyphenoxy, 4-Methoxyphenoxy, 2,4-Dimethoxyphenoxy, 3,4,5-Trimethoxyphenoxy, 2-Chlorbenzyl, 2,3-Dichlorbenzyl, 2-Methylbenzyl, 2-Trifluormethylbenzyl, 4-Methoxybenzyl, 3,4,5-Trimethoxybenzyl, 2-(2-Chlorphenyl)ethyl, 2-Chlorbenzyloxy, 2,4-Dimethylbenzyloxy, 2-Trifluormethylbenzyloxy, 3,4,5-Trimethoxybenzyloxy, 2-(2-Chlorophenyl)ethoxy, 2-(2,4-Dimethylphenyl)ethoxy.

Eine Phenylgruppe kann als weiteren Substituenten auch eine über eine $C_1$ - $C_4$-Alkylenkette - bevorzugt Ethylenkette, $-OCH_2$, oder $-CH_2-O-CH_2$-verknüpfte Phenylgruppe tragen, wobei diese in Analogie zu den oben aufgeführten Phenylresten definiert sein kann. Beispiele für gegebenenfalls substituierte gesättigte oder ungesättigte heterocyclische 5-, 6- oder 7-gliedrige Ringe bzw. Heteroarylreste sind:

Pyrrol, Pyrrolin, Pyrrolidin, 2-Methylpyrrolidin, 3-Methylpyrrolidin, Piperidin - gegebenenfalls durch $C_1$-$C_4$ Alkyl ein- oder mehrfach substituiert -Piperazin, N-Methylpiperazin, N-Ethylpiperazin, N-n-Propylpiperazin, N-Benzylpiperazin, Morpholin, Thiomorpholin, Imidazol, Imidazolin, Imidazolidin, Triazol, Pyrazol, Pyrazolin, Pyrazolidin, Triazin, 1, 2, 3, 4 - Tetrazin, 1, 2, 3, 5 - Tetrazin, 1, 2, 4, 5 - Tetrazin - wobei die genannten Heterocyclen durch Alkyl mit 1 bis 4 Kohlenstoffatomen - bevorzugt Methyl - substituiert sein können;

Als heterocyclische Reste, die über ein Kohlenstoffatom verknüpft sein können, werden beispielsweise Thiophen, 2-Methylthiophen, Furan, Tetrahydrofuran, 2-Methyltetrahydrofuran, Tetrahydrofuran, 2-Hydroxymethylfuran, $\alpha$-Pyran, $\gamma$-Pyran, 1,3-Dioxolan, 1,2-Oxathiolan, 1,2-Oxathiepan, Tetrahydro-pyran, Thiolan, 1,3-Dithian, 1,3-Dithiolan, 1,3-Dithiolen, genannt, wobei der Heterocyclus durch $C_1$-$C_4$ Alkyl, $C_1$-$C_4$ Alkoxy, Halogen substituiert sein kann.

Als Heterocyclus im Rahmen der zuvor angegebenen Definition steht im allgemeinen für einen 5- bis 6-gliedrigen Ring, der als Heteroatome Sauerstoff, Schwefel und/oder Stickstoff enthalten kann und an den ein weiterer aromatischer Ring bevorzugt ein Phenylring ankondensiert sein kann. Bevorzugt sind 5- und 6-gliedrige aromatische Ringe, die einen Sauerstoff, einen Schwefel und/oder bis zu zwei Stickstoffatomen enthalten und die gegebenenfalls benzokondensiert sind. Als besondere Heteroarylreste seien beispielsweise Thienyl, Furyl, Pyridyl, Pyrimidyl, Pyrazinyl, Pyrazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl genannt.

Der Heterocyclus kann durch Halogen, Hydroxy und/oder verzweigtes oder unverzweigtes Alkyl mit 1 bis 4 Kohlenstoffatomen verzweigtes oder unverzweigtes Alkoxy mit 1 bis 4 Kohlenstoffatomen substituiert sein.

Bevorzugte Imidoreste sind die folgenden Strukturen

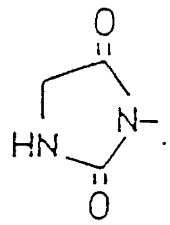

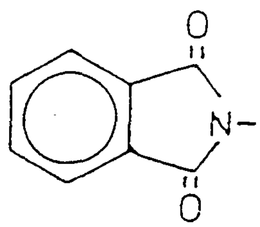

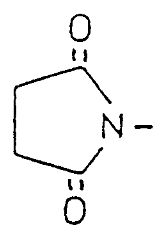

Verbindungen der allgemeinen Formel I, worin $R_5$ H ist, weisen an diesem Kohlenstoffatom ein Asymmetriezentrum auf.

Überraschenderweise wurde gefunden, daß die (-)-Enantiomeren der allgemeinen Formel I - insbesondere wenn $R_5$ = Methyl bedeutet, gegenüber den (+)-Enantiomeren eine wesentlich stärkere pharmakologische Wirksamkeit aufweisen. Die (-)-Enantiomeren der allgemeinen Formel gelten daher als bevorzugte Verbindungen und werden als solche beansprucht. Diese Aussage bezieht sich auf solche Enantiomere, deren $[\alpha]^D 20$ Wert [c = 1, Methanol] einen negativen Wert annimmt.

Die bei der Synthese im allgemeinen erhaltenes Racemat kann durch präparative Verfahren - z.B.Säulenchromatographie - in die optisch reinen Enantiomere aufgetrennt werden. Ein geeignetes Säulenmaterial ist beispielsweise Poly-N-acryloyl-L-phenylalaninethylester, wobei ein Gemisch von n-Hexan und Dioxan 60/40 als Elutionsmittel verwendet werden kann.

Die erfindungsgemäßen Verbindungen können bevorzugt gemäß dem folgenden Verfahrensschema hergestellt werden.

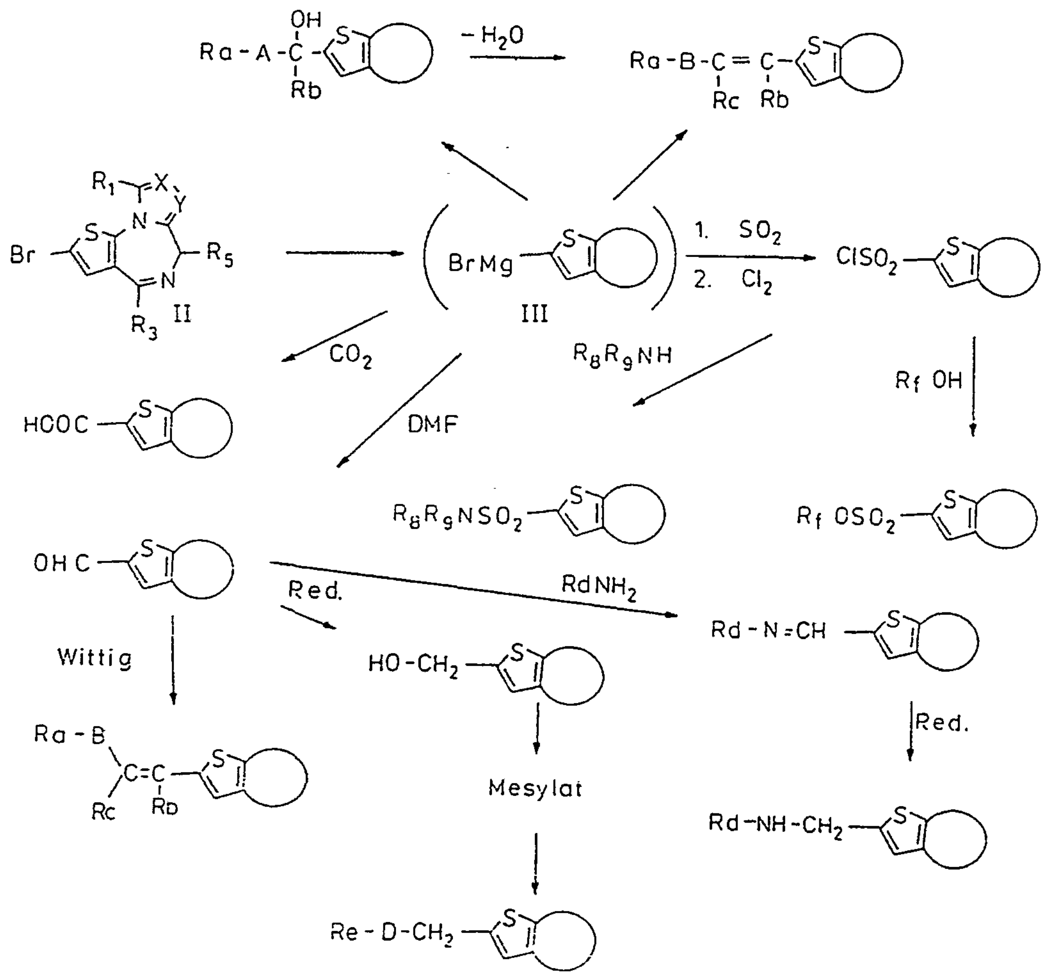


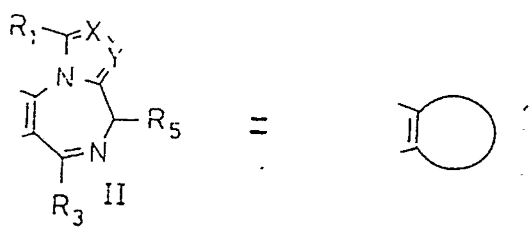


A bevorzugt - $(CH_2)_n$

B bevorzugt - $(CH_2)_m$

Wie im Syntheseschema angegeben, werden Thienodiazepine der allgemeinen Formel II einer Umgrignardierung unterworfen. Die Umsetzung kann mit bekannten - einfach zugänglichen Grignardverbindungen der allgemeinen Formel $A^xMg$ Hal, worin $A^x$ ein geeigneter organischer Rest und Hal ein Halogenid, wie z. B. Brom oder Jod ist, in inerten organischen Lösungsmitteln durchgeführt werden. Bevorzugte Grignardverbindungen sind Ethylmagnesiumbromid, Ethylmagnesiumjodid, Methylmagnesiumbromid, Methylmagnesiumjodid und Phenylmagnesiumbromid. Bevorzugtes Lösungsmittel ist Tetrahydrofuran, andere Lösungsmittel sind geeignet, soweit sich die Ausgangsverbindungen darin lösen.

Alternativ können Verbindungen der allgemeinen Formel II anstelle mit den zuvor beschriebenen Grignardverbindungen auch mit anderen metallorganischen Reagenzien - insbesondere mit Alkaliorganylen, wie zum Beispiel n-Butyllithium, tert-Butyllithium, Phenyllithium, Methyllithium, umgesetzt werden. Auch hierbei sind zwei Äquivalente des metallorganischen Reagenzes auf ein Äquivalent einer Verbindung der allgemeinen Formel II einzusetzen, um eine reaktive Zwischenverbindung III zu erhalten.

Das durch die Umsetzung der Bromverbindung der allgemeinen Formel II und zweier Äquivalente der oben genannten Grignardverbindung, bzw. einer metallorganischen Verbindung erhaltene Zwischenprodukt der allgemeinen Formel III wurde nicht näher charakterisiert. Aus diesem Grund ist unter der Definition der allgemeinen Formel III im Formelbild das Umsetzungsprodukt von II mit einem metallorganischen Reagenz - bevorzugt mit einem Grignardreagenz in einem inerten organischen Lösungsmittel zu verstehen. Dieses wird als solches als Ausgangsprodukt zur Herstellung anderer Thienodiazepine beansprucht.

Im allgemeinen wird die Umsetzung bei Temperaturen zwischen -10° C und +40° C - bevorzugt zwischen 0° C und 20° C durchgeführt.

Die so erhaltene metallorganische Verbindung der allgemeinen Formel III wird mit geeigneten elektrophilen Reagenzien weiter umgesetzt, die so erhaltenen Verbindungen werden gegebenenfalls in nachgeschalteten Umsetzungen in Verbindungen der allgemeinen Formel I überführt.

Carbinole der allgemeinen Formel I erhält man durch Umsetzung der Grignardzwischenstufe III mit Aldehyden und Ketonen. Durch Dehydratisierung dieser Carbinole werden Verbindungen der allgemeinen Formel I erhalten, worin $R_2$ = $R_a$ -B-$CR_c$ = $CR_c$ -,bevorzugt $R_2$ = $R_a$-$(CH_2)_m$CH-$CR_b$ bedeutet. Alternativ kann die Doppelbindung durch Umsetzung der der magnesiumorganischen Verbindung II im Sinne einer Palladium katalytisierten Wurtz-Reaktion -beispielsweise mit Tetrakis-(triphenylphosphin)palladium - eingeführt werden.

Sulfonester und Sulfonamide erhält man durch Umsetzung der Grignardzwischenstufe mit $SO_2$ in inerten organischen Lösungsmitteln bei Raumtemperatur. Das dabei entstehende Sulfinsäuresalz wird nach Entfernen des Lösungsmittels ohne weitere Reinigung weiter umgesetzt. Lösen in einem System aus einem chlorierten Kohlenwasserstoff - wie z.B. Dichlormethan - und Wasser und anschließendes Einleiten von Chlor ergibt das entsprechende Sulfonsäurechlorid, das mit Aminen bzw. Alkoholen zu den entsprechenden Sulfonsäureamiden bzw. Sulfonsäureestern umgesetzt werden kann.

Die Umsetzung einer Grignardverbindung der allgemeinen Formel III mit Dimethylformamid in Tetrahydrofuran bei Raumtemperatur führt zu Thienodiazepinen worin $R_2$ -CH=O bedeutet.

Verbindungen der allgemeinen Formel I worin $R_2$ die Bedeutung eines Aldehyds aufweisen sind wertvolle Zwischenprodukte zur Synthese weitere Derivate und werden als solche beansprucht.

Unter den Reaktionsbedingungen einer Wittig- bzw. Wittig-Horner-Reaktion wird ausgehend vom Aldehyd eine durch eine Doppelbindung verknüpfte Seitenkette in das Molekül eingeführt.

Durch Umsetzung des Aldehyds mit primären Aminen gelangt man zu Schiffschen Basen, die anschließend zu sekundären Aminen reduziert werden können.

Ausgehend von Aldehyd ($R_2$ = -CH=O) erhält man durch Reduktion - beispielsweise mit Natriumborhydrid in Methanol- den entsprechenden Alkohol. Dieser kann in das Mesylat überführt werden, das als wichtiges Zwischenprodukt mit nucleophilen Reagenzien umesetzt werden kann. Unter Anwendung dieser Reaktion können Ether und Thioeteher des Typs $R_2$ = $R_e$-A-$CH_2$ synthetisiert werden.

Nach an sich bekannten Verfahren können Verbindungen der allgemeinen Formel Ia, worin $R_2$ einen Ester -bevorzugt einen Methyl- oder Ethylester - enthält zur Carbonsäure verseift werden. Ausgehend von Verbindungen der allgemeinen Formel III erhält man durch Umsetzung mit $CO_2$ in inerten organischen Lösungsmitteln Verbindungen der allgemeinen Formel Ia worin $R_2$ den Rest COOH bedeutet. Verbindung der allgemeinen Formel Ia worin $R_2$ ein ungesättigtes Säureamid darstellt, erhält man durch Wittig-Horner-Olefinierung von Verbindung der allgemeinen Formel Ia worin $R_2$ eine Aldehydfunktion enthält.

Verbindungen der allgemeinen Formel Ib können ebenfalls nach an sich bekannten Verfahren durch Reduktion, Alkylierung oder Acylierung von Verbindungen der allgemeinen Formel Ia erhalten werden. Gegebenenfalls sind hierbei funktionelle Gruppen durch abspaltbare Schutzgruppen zu schützen. Einzelheiten nachgeschalteter Umsetzungen können beispielsweise der europäischen Patentanmeldung 0230 942 entnommen werden.

Die erfindungsgemäßen Verbindungen können nach an sich bekannten Verfahren in ihre Säureadditionssalze überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Essigsäure, Propionsäure, Buttersäure, Capronsäure, Valeriansäure, Oxalsäure, Malonsäure, Bernsteinsäure, Maleinsäure, Fumarsäure, Milchsäure,

Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen. Bevorzugte Säureadditionssalze sind die Hydrochloride sowie Hydrobromide.

Die erfindungsgemäßen Verbindungen besitzen PAF-antagonistische Wirkung.
Bekanntlich handelt es sich bei PAF (Plättchen Aktivierender Faktor) um das Phospholipid Acetyl-glyceryl-ether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.
Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für mögliche Indikationen eines PAF-Antagonisten sind Entzündungsprozesse des Tracheobronchialbaumes (akute und chronische Bronchitis, Asthma bronchiale) oder der Niere (Glomerulonephritis), der Gelenke (rheumatische Erkrankungen), anaphylaktische Zustände, Allergien und Entzündungen im Bereich der Schleimhäute (Rhinitis, Konjunktivitis) und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Schockulcus, Colitisulcerose, Morbus Crohn, Stressulcus, im allgemeinen Ulcus pepticum, jedoch insbesondere Ulcus ventriculi und Ulcus duodeni;

Obstruktive Lungenerkrankungen, wie z.B. bronchiale Hyperreaktivität, entzündliche Lungenwegserkrankungen, wie z.B. chronische Bronchitis;
Herz- Kreislauferkrankungen, wie z.B. Polytrauma, Anaphylaxe, Arteriosklerose, entzündliche Darmerkrankungen, EPH-Gestose (ederma-proteinuria Hypertension), Erkrankungen des extrakorporalen Kreislauf z.B. Herzinsuffizienz, Herzinfakt, ischämische Erkrankungen, entzündliche und immunologische Erkrankungen, Immunmodulation bei Transplantationen von Fremdgeweben, Immunmodulation bei Leukämie. Metastasenausbreitung z.B. bei bronchialer Neoplasie, Erkrankungen des ZNS, wie z.B. Migräne, Agarophobie (panic disorder), weiterhin erweisen sich die erfindungsgemäßen Verbindungen als Cyto- und Organoprotektiv, z.B. zur Neuroprotektion, z.B. bei Leberzirrhose, DIC (disiminierte intravasale Gerinnung);

Nebenwirkungen einer Arzneimitteltherapie, z.B. anaphylaktoide Kreislaufreaktionen, Kontrastmittelzwischenfälle, Nebenwirkungen bei der Tumortherapie;
Unverträglichkeiten bei Bluttransfusionen; fulminantes Leberversagen ($CCl_4$-Intoxikation) Amanitaphalloides-Intoxikation (Knollenblätterpilzvergiftung);
Symptome von parasitären Erkrankungen (z.B. Wurmerkrankungen); Autoimmunerkrankungen; Autoimmunhaemolytischen Anaemien, autoimmunologisch bedingte Glomerulonephritiden, Thyreoidis Hashimoto, primäres Myxoedem, perniziöse Anaemie, autoimmune atrophische Gastritis, Morbus Addison, iuveniler Diabetes, Goodpasture-Syndrom, idiopathische Leukopenie, primär biliäre Zirrhose, aktive bzw. chronisch aggressive Hepatitis (HBsAg-neg.), Colitis ulcerosa und systemischer Lupus erythematodes (SLE), ideopathische thrombrozytopenische Parpura (ITP).

Immunfunktion bei Aids, Diabetes, juvenile Diabetes, diabetische Retinopathie, polytraumatischer Schock, hämorrhagischer Schock, CNS: Ischämie, Multiple Sklerose, endogene Depression.
PAF assoziierte Interaktion mit Gewebshormon (autocoid hormones), Lymphokine und anderen Mediatoren.

In Bindungsstudien zeigen die erfindungsgemäßen Verbindungen eine signifikante Bindung an den PAF-Rezeptor mit [$^3$H]-PAF als Radioligand und eine relativ schwache Bindung im Benzodiazepin - Rezeptor Bindungstest mit [$^3$H] Flunitrazepam als Radioligand (Bechtel et al. in Arzneimittelforschung 3a, 1986, 534). So zeigt beispielsweise das 4-(2-Chlorphenyl)-6H-9-methyl-2-[1-(3,4-dichlorphenyl)-1-hydroxy-methyl]-thieno-[3 2-f]-[1,2,4]triazolo[4,3-a][1,4]diazepin einen $IC_{50}$Wert von 313 $\cdot 10^{-9}$ Mol/ltr. und das 4-(2-Chlorphenyl)-6H-9-methyl-2[1-(4-trifluormethylphenyl)-1-hydroxy-methyl]-thieno[3,2-f][1,2,4]triazolo[4,3-a]-[1,4]-diazepin einen $IC_{50}$-Wert von 241 $\cdot 10^{-9}$ Mol/ltr.; ein Indiz dafür, daß die erfindungsgemäßen Verbindungen nicht mehr zentral wirken.

Beispiele

Beispiel 1

4-(2-Chlorphenyl)-6H-9-methyl-2-[1-(4-methylphenyl)-1-hydroxy-methyl]-thieno[3.2-f][1.2.4]-triazolo[4.3-a]-[1.4]diazepin

In 5 ml absolutem Tetrahydrofuran wird aus 1.6 g Bromethan und 0.5 g Magnesium eine Ethylmagnesiumbromid-Lösung bereitet und tropfenweise mit 4.0 g 2-Brom-4-(2-chlorphenyl)-6H-9-methyl-thieno-[3.2-f] [1.2.4]triazolo-[4.3-a][1.4]diazepin = Brotizolam = Lendormin$^R$, warm gelöst in 70 ml absolutem Tetrahydrofuran versetzt. Nach 2 Stunden Rühren tropft man 2.3 ml 4-Methylbenzaldehyd bei Raumtemperatur zu und rührt über Nacht. Das Reaktionsgemisch wird mit 25 ml einer gesättigten Ammoniumchloridlösung versetzt und von der organischen Phase nach dem Trocknen das Solvens abgezogen. Der Rückstand läßt sich aus Essigester umkristallisieren und ergibt in 74 % Ausbeute 3.2 g der Titelverbindung vom Fp. 209-210 °C.

(Alternativ kann Brotizolam mit 2 Äquivalenten n-Butyllithium metalliert werden (Tetrahydrofuran, 0 °C). Die weitere Umsetzung erfolgt analog Beispiel 1.)

Beispiel 2

4-(2-Chlorphenyl)-2-(3.4-dimethoxyphenoxysulfonyl)--6H-9-methyl-thieno[3.2f][1.2.4]triazolo[4.3-a]-[1.4]-diazepin

Aus 4.8 g Bromethan, 1.5 g Magnesium und 12 g Brotizolam wird, wie unter 1 beschrieben, die Grignard-Zwischenstufe hergestellt, die Tetrahydrofuranlösung auf 0 °C gekühlt und 30 min. Schwefeldioxid eingeleitet. Vom Reaktionsgemisch wird das Solvens abgezogen, der Rückstand in Wasser/Methylenchlorid aufgenommen und unter 2-Phasenbedingungen unter Eiskühlung 30 Minuten Chlor-Gas eingeleitet, die organische Phase abgetrennt, getrocknet und das Solvens abgezogen.

Zu einer Suspension von 2.6 g des so erhaltenen Sulfochlorid-Rohprodukts in 20ml absolutem Tetrahydrofuran tropft man eine Suspension, hergestellt aus 1.5 g 3,4-Dimethoxyphenol und 0.5 g NaH-Dispersion in 20 ml absolutem Tetrahydrofuran, zu. Nach Zugabe von 0.1 Mol Dimethylformamid (DMF) wird 2 Stunden bei Raumtemperatur gerührt, das Reaktionsgemisch mit etherischer Salzsäure auf pH 7-8 eingestellt, und im Vakuum eingeengt. Den Rückstand nimmt man in Methylenchlorid/Wasser auf, wäscht die organische Phase mit verdünnter Natronlauge und Wasser und chromatographiert nach dem Abdestillieren des Solvens den Rückstand an Kieselgel mit Methylenchlorid: Methanol 9:1 als Eluens. Man erhält in 27 % Ausbeute 0.9 g der Titelverbindung.

Beispiel 3

4-(2-Chlorphenyl)-6H-9-methyl-2-morpholinosulfonyl-thieno-[3.2-f][1.2.4]triazolo[4.3-a][1.4]diazepin

Zu einer Suspension von 4.0 g des in Beispiel 2 beschriebenen Sulfochlorid-Rohprodukts in 80 ml absolutem Methylenchlorid tropft man bei Raumtemperatur 2.6 ml Morpholin zu. Die Suspension wird mit Wasser, gesättigter $NaHCO_3$-Lösung und erneut mit Wasser gewaschen und nach dem Abdestillieren des Solvens der Rückstand an Kieselgel mit Methylenchlorid: Methanol 9:1 als Eluens chromatographiert. Man erhält in 21 % Ausbeute 1.0 g des Sulfonamids.

Beispiel 4

4-(2-Chlorphenyl)-2-formyl-6H-9-methyl-thieno[3.2-f][1.2.4]triazolo[4.3-a][1.4]diazepin

Aus 4 g Brotizolam, 0.5 g Magnesium und 1.6 ml Bromethan wird in 75 ml Tetrahydrofuran, wie in Beispiel 1 beschrieben, die Grignardlösung hergestellt, zu der 1.5 ml DMF bei Raumtemperatur zugetropft

werden. Nach 2 Stunden Rühren zieht man das Solvens ab, flash-chromatographiert (Mitteldruck-Flüssigkeits-Chromatographie) den Rückstand an Kieselgel mit Methylenchlorid: Methanol = 9:1 als Eluens und kristallisiert anschließend aus Acetonitril um. Der Aldehyd vom Fp. 186-187° C wird in 52 % Ausbeute erhalten.

Beispiel 5

2-Carboxy-4-(2-chlorphenyl)-6H-9-methyl-thieno[3.2-f][1.2.4]triazolo[4.3-a][1.4]diazepin

Aus 4 g Brotizolam, 0,5 g Magnesium und 1.6 ml Bromethan wird in 75 ml Tetrahydrofuran, wie in Beispiel 1 beschrieben, die Grignardlösung hergestellt, in die man 30 min $CO_2$ einleitet. Nach 2 Stunden Rühren wird das Reaktiongemisch mit gesättigter Ammoniumchloridlösung hydrolysiert, die organische Phase abgetrennt und das Solvens abgezogen. Den Rückstand nimmt man in Methylenchlorid auf und gibt das gleiche Volumen verdünnte Natronlauge zu, trennt die wäßrige Phase ab, wäscht mit Methylenchlorid nach und säuert die alkalische wäßrige Phase mit verdünnter Salzsäure an. Die ausgefallene Carbonsäure wird abgesaugt und mit Ether nachgewaschen. Fp. 302° C, Ausbeute 71 %.

Beispiel 6

4-(2-Chlorphenyl)-6H-9-methyl-2-(2-phenyl-ethen-1-yl)-thieno[3.2-f][1.2.4]triazolo[4.3-a][1.4]diazepin

Analog Beispiel 1 werden unter Inertgas in 50 ml absolutem Tetrahydrofuran 2.95 g Brotizolam in die magnesiumorganische Verbindung überführt und zu einer Lösung von 3.4 g 80 %igem Bromstyrol und 0.5 g Tetrakis-(triphenylphosphin)-palladium in 30 ml Benzol, die eine Stunde bei Raumtemperatur gerührt wurden, zugetropft. Das Reaktionsgemisch rührt man 48 Stunden bei Raumtemperatur weiter, zersetzt anschließend mit Ammoniumchloridlösung, dampft die organische Phase nach dem Trocknen ein, flash-chromatographiert den Rückstand an Kieselgel mit Methylchlorid : Methanol (9 : 1) als Eluens und erhält 0.9 g der Verbindung 6 in 29 % Ausbeute als Öl.

Alternativ kann die Verbindung 6 wie folgt hergestellt werden:

1.6 g 4-(2-Chlorphenyl)-6H-2-[(1-hydroxy-2-phenyl)-ethyl]-9-methyl-thieno[3.2-f][1.2.4]triazolo[4.3-a][1.4]-diazepin, 1.6 g Phosphorsäure und 8 g Kieselgel werden in 50 ml Toluol am Wasserabscheider 10 Stunden zum Rückfluß erhitzt. Das Kieselgel wird abgesaugt und mehrmals mit Methanol gewaschen. Man dampft i. Vak das Methanolfiltrat ein, chromatographiert den Rückstand an Kieselgel mit Methanol: Methylenchlorid = 1:9 als Eluens und erhält die Verbindung 6 in 39 % Ausbeute als Öl.

Beispiel 66

4-(2-Chlorphenyl)-6H-9-methyl-2-(2-morpholinocarbonyl-ethen-1-yl)-thieno[3.2-f][1.2.4]triazolo[4.3-a][1.4]-diazepin

0.9 g (3.3 mmol) Diethylphosphonoessigsäuremorpholid wird mit 0.16 g 55 %iger Natriumhydrid-Dispersion in 15 ml absolutem Tetrahydrofuran in das Anion überführt und noch 30 Minuten bei Raumtemperatur weitergerührt. Anschließend tropft man 1.0 g (3.0 mmol) des Beispiels 4 in 15 ml absolutem Tetrahydrofuran bei Raumtemperatur zu, rührt zwei Stunden weiter und zieht anschließend das Solvens ab. Der Rückstand wird in Methylenchlorid/Wasser aufgenommen und die organische Phase gewaschen, getrocknet, das Solvens abgezogen und der Rückstand an Kieselgel zweimal mittels Flash-Chromatographie gereinigt, zunächst mit Methylenchlorid : Methanol (9 : 1) als Eluens, anschließend mit Aceton : Methanol (9 : 1) als Eluens. Aus Essigester umkristallisiert erhält man in 37 % Ausbeute 0.5 g der Verbindung 66 mit Fp. 241 - 243° C.

Beispiel 76

4-(2-Chlorphenyl)-6H-9-methyl-2-(N-phenyl-aminomethyl)-thieno[3.2-f][1.2.4]triazolo[4.3-a][1.4]diazepin

3.4 g des Beispiels 4, 1.4 ml Anilin und 0.1 g p-Toluolsulfonsäure werden in 100 ml Tetrahydrofuran eine Stunde bei Raumtemperatur gerührt, dann zwei Stunden zum Rückfluß erhitzt und anschließend das Solvens im Vakuum abgezogen. Der Rückstand wird aus Acetonitril umkristallisiert und ergibt in 64 % Ausbeute 2.7 g des Azomethins, Beispiel 72, vom Fp. 231 - 232° C.

1 g des Azomethins wird in 60 ml Methanol bei Raumtemperatur mittels Raney-Nickel als Katalysator bei 5 bar innerhalb drei Stunden hydriert. Anschließend saugt man den Katalysator ab, zieht das Solvens ab und kocht den zurückbleibenden Kristallbrei mit Acetonitril aus. Man erhält so in 42 % Ausbeute das Beispiel 76 vom Fp. 245 - 247° C.

Beispiel 80

4-(2-Chlorphenyl)-6H-9-methyl-2-(4-tert.-butylphenoxy-methyl)-thieno[3.2-f][1.2.4]triazolo[4.3-a][1.4]diazepin

Ausgehend von Beispiel 4 erhält man durch Reduktion mit Natriumborhydrid in Methanol die Verbindung 71, die in absolutem Methylenchlorid mit Methansulfonsäurechlorid und Triethylamin als Säurefänger bei Raumtemperatur in das Mesylat umgewandelt wird.

Aus 3 g 4-tert.-Butylphenol und der equivalenten Menge Natriumhydrid-Dispersion bereitet man sich in 40 ml Dioxan die entsprechende Phenolatlösung, die noch 30 Minuten nachgerührt wird. Zu dieser Lösung tropft man bei Raumtemperatur 3.8 g des obigen Mesylats in 40 ml Dioxan gelöst zu und erhitzt das Reaktionsgemisch zwei Stunden zum Rückfluß. Nach dem Abziehen des Solvens wird der Rückstand in Methylenchlorid/Wasser aufgenommen, die organische Phase mit gesättigter Kochsalzlösung gewaschen und nach dem Abziehen des Solvens der Rückstand an Kieselgel flash-chromatographiert mit Methylenchlorid/Methanol (95 : 5) als Eluens. Die so gereinigte Verbindung wird aus Ethanol umkristallisiert und ergibt in 36 % Ausbeute 1.8 g des Beispiels 80 vom Fp. 173 - 175° C.

Auf analoge Weise können die nachfolgenden Verbindungen erhalten werden.

Tabelle I

Verbindungen Ia mit X,Y=N, wenn nicht anders vermerkt.

| Nr. | $R_1$ | $R_5$ | $R_3$ | $R_2$ | Fp/NMR |
|---|---|---|---|---|---|
| 7 | Me | H | 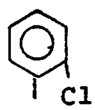 | 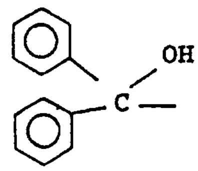 | 249-250 |
| 8 | Me | H | " | 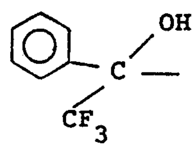 | 170-172 |
| 9 | Me | H | " | $n-C_3H_7$ | |
| 10 | Me | H | " | $n-C_8H_{17}$ | |
| 11 | Me | H | " | 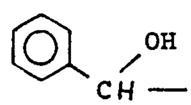 | 221-224 |
| 12 | 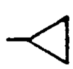 | $CH_3$ | " | " | |

| Nr. | $R_1$ | $R_5$ | $R_3$ | $R_2$ | Fp/NMR |
|---|---|---|---|---|---|
| 13 | MeO | H | 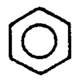 | 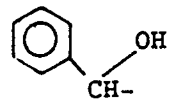 | |
| 14 | Me | H | 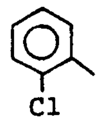 | 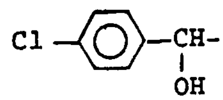 | 219-220 |
| 15 | Me | H | " | 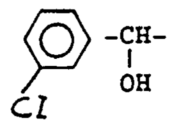 | amorph |
| 16 | Me | H | " | 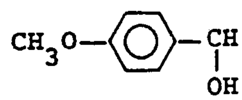 | 216-217 |
| 17 | Me | OH | " | " | |
| 18 | Me | -OAc | " | " | |
| 19 | Me | H | " | 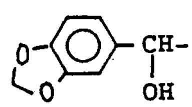 | 206-208 |
| 20 | Me | H | " | 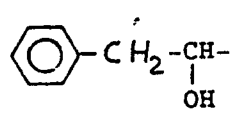 | amorph |

| Nr. | $R_1$ | $R_5$ | $R_3$ | $R_2$ | Fp/NMR |
|---|---|---|---|---|---|
| 21 | Me | H | 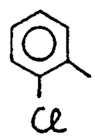 | 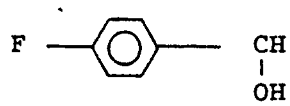 | 242-244 |
| 22 | Me | H | " | 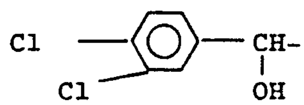 | 115-119 |
| 23 | Me | H | " | 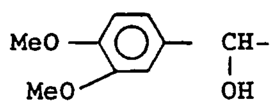 | 164-165 |
| 24 | Et | H | " | " | |
| 25 | Et | H | 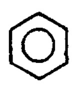 | " | |
| 26 | Me | H | 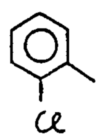 | 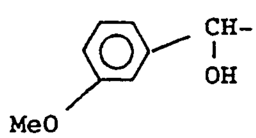 | 172-174 |
| 27 | Me | H | " | 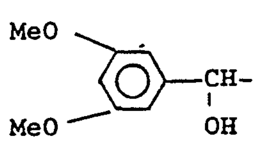 | 178-180 |
| 28 | Me | H | " | 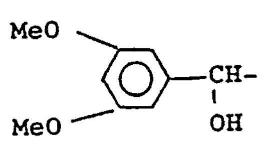 | |

X=CH

| Nr. | $R_1$ | $R_5$ | $R_3$ | $R_2$ | Fp/NMR |
|---|---|---|---|---|---|
| 29 | Me | H | 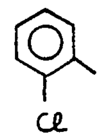 | 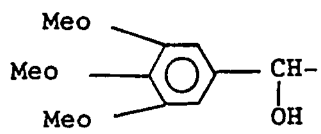 | 213-214 |
| 30 | Me | H | " | 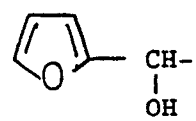 | 94-95 |
| 31 | Et | H | " | 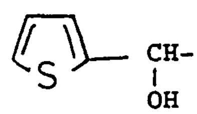 | |
| 32 | Me | H | 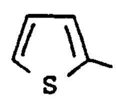 | 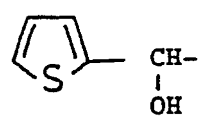 | |
| 33 | $CH_3$ | H | 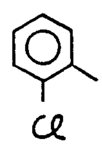 | 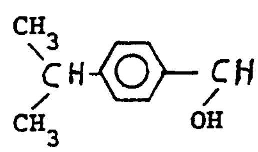 | 218-219 |
| 34 | $CH_3$ | H | " | 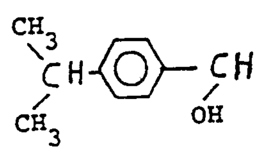 X=CH | |
| 35 | $CH_3$ | H | " | 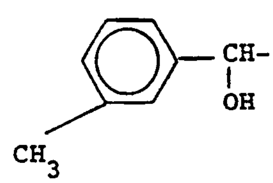 | 114 |

| Nr. | $R_1$ | $R_5$ | $R_3$ | $R_2$ | Fp/NMR |
|---|---|---|---|---|---|
| 36 | $CH_3$ | H | 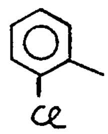 | 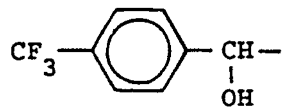 | 214-218 |
| 37 | $CH_3$ | H | " | 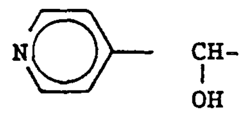 | |
| 38 | $CH_3$ | H | " | 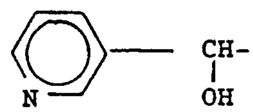 | |
| 39 | $CH_3$ | H | " | 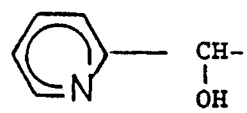 | |
| 40 | $CH_3$ | H | " | 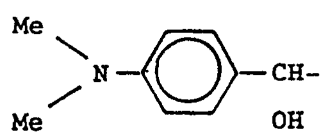 | 55 |
| 41 | $CH_3$ | H | " | 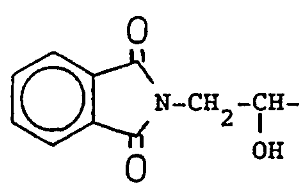 | 172-175 |
| 42* | $CH_3$ | H | " | $NH_2-CH_2-CH-$ OH | Öl |

* hergestellt aus 41 mittels Hydrazinspaltung

| Nr. | $R_1$ | $R_5$ | $R_3$ | $R_2$ | Fp/NMR |
|---|---|---|---|---|---|
| 43* | $CH_3$ | H | 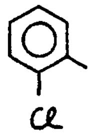 | 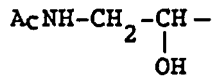 | Öl |
| 44 | $CH_3$ | H | 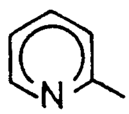 | 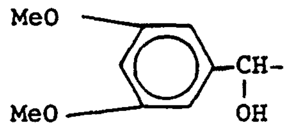 | |
| 45 | $CH_3$ | H | 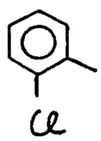 | $CH_3-O-SO_2-$ | |
| 46 | $CH_3$ | H | " | 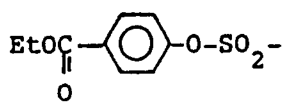 | 110-112 |
| 47 | $CH_3$ | H | " | 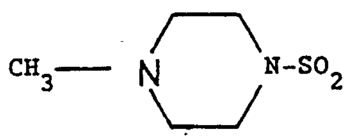 | Öl |

* hergestellt durch Acylierung von 42

| Nr. | $R_1$ | $R_5$ | $R_3$ | $R_2$ | Fp/NMR |
|---|---|---|---|---|---|
| 48 | $CH_3$ | H | 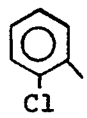 | $CH_3OC-CH_2-NH-SO_2$<br>O | öl |
| 49 a) | $CH_3$ | H | " | $HOOC-CH_2-NH-SO_2-$ | öl |
| 50 | $CH_3$ | H | " | $MeOC-CH_2-CH_2-NH-SO_2$<br>O | |
| 51 a) | $CH_3$ | H | " | $HO-C-CH_2-CH_2-NH-SO_2$<br>O | |
| 52 | $CH_3$ | H | " | 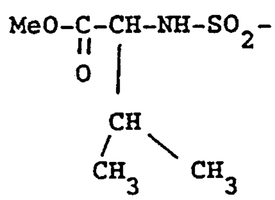 | |
| 53 a) | $CH_3$ | H | " | $HO-C-CH-NH-SO_2-$<br>O<br>$HC(CH_3)_2$ | |
| 54 | $CH_3$ | H | " | 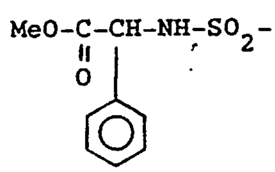 | |

| Nr. | $R_1$ | $R_5$ | $R_3$ | $R_2$ | Fp/NMR |
|---|---|---|---|---|---|
| 55 a) | $CH_3$ | H | 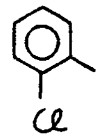 | 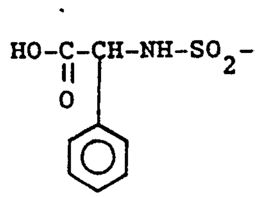 | |
| 56 | $CH_3$ | H | " | 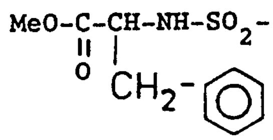 | |
| 57 a) | $CH_3$ | H | " | 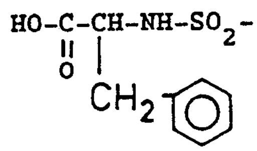 | |
| 58 | $CH_3$ | H | " | 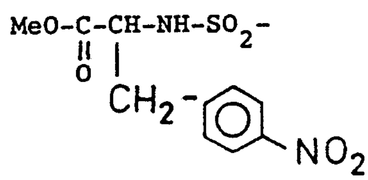 | |
| 59 a) | $CH_3$ | H | " | 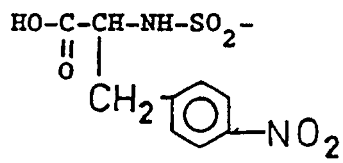 | |
| 60 | $CH_3$ | H | " | 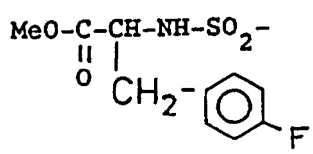 | |
| 61 a) | $CH_3$ | H | " | 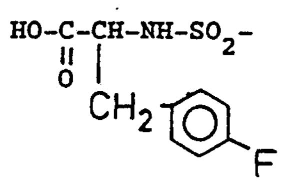 | |

| Nr. | $R_1$ | $R_5$ | $R_3$ | $R_2$ | Fp/NMR |
|---|---|---|---|---|---|
| 62 | $CH_3$ | H | 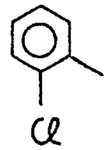 | 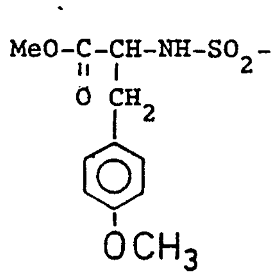 | |
| 63 a) | $CH_3$ | H | " | 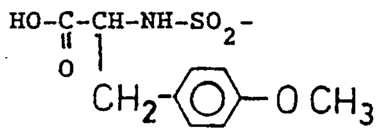 | |
| 64 | $CH_3$ | H | " | 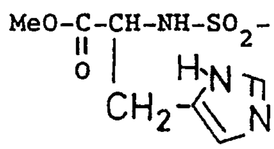 | |
| 65 | $CH_3$ | H | " | 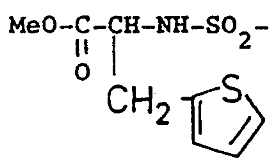 | |
| 66 | $CH_3$ | H | " | 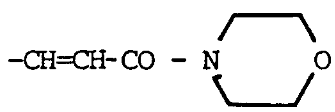 | 241-243 |

a) Die mit a) gekennzeichneten freien Sulfonamidocarbonsäuren werden durch alkalische Verseifung der entsprechenden Ester hergestellt.

| Nr. | $R_1$ | $R_5$ | $R_3$ | $R_2$ | Fp/NMR |
|---|---|---|---|---|---|
| 67 | $CH_3$ | H | 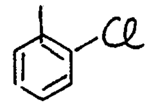 | 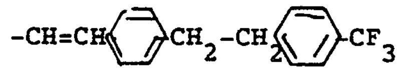 | 185 - 186°C |
| 68 | $CH_3$ | " | " | 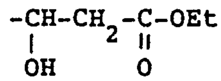 | Öl[a)] |
| 69 | $CH_3$ | " | " | 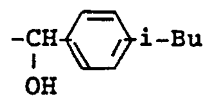 | 218°C |
| 70 | $CH_3$- | | " | 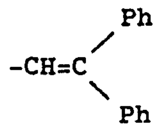 | Öl |
| 71 | " | " | " | $-CH_2-OH$ | Öl[b)] |
| 72 | " | " | " | -CH=N-Ph | 231-232°C |
| 73 | " | " | " | 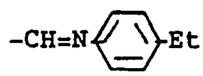 | 233-235°C |
| 74 | " | " | " | 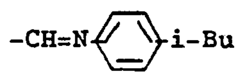 | |
| 75 | " | " | " | 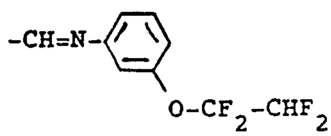 | 156-157°C |
| 76 | " | " | " | $-CH_2-NH-Ph$ | 245-247°C |
| 77 | " | " | " | 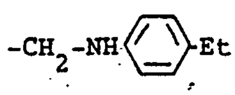 | Öl |
| 78 | " | " | " | 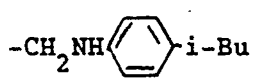 | Öl |

a) hergestellt aus Beispiel 4 durch Reformatzky-Reaktion (Zn, $Br-CH_2-COOEt$)

b) hergestellt aus Beispiel 4 durch Reduktion mit $Na\,BH_4$/Methanol)

| Nr. | $R_1$ | $R_5$ | $R_3$ | $R_2$ | Fp/NMR |
|---|---|---|---|---|---|
| 79 | $CH_3$ | H | 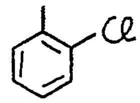 | 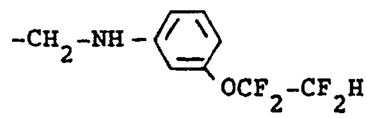 | Öl |
| 80 | " | " | " | 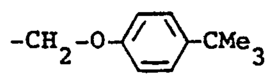 | 173-175°C |
| 81 | " | " | " | 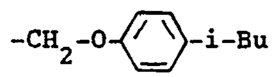 | |
| 82 | " | " | " | 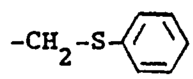 | Öl[d)] |
| 83 | " | " | " | 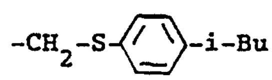 | Öl[d] |
| 84 | " | " | " | 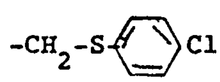 | Öl[d] |
| 85 | $CH_3$- | $CH_3$ | | 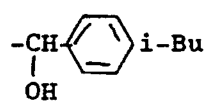 | Öl |
| 86 | $CH_3$- | EtO-C- | | 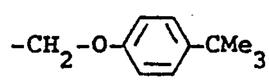 | Öl[c)] |
| 87 | $CH_3$- | $HOCH_2$- | | 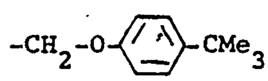 | Öl[b)] |

b) hergestellt aus Beispiel 86 durch Reduktion mit $NaBH_4$/Methanol

c) hergestellt aus Beispiel 80 mittels NaH/$(EtO)_2C=O$

Tabelle II: NMR-Daten

Beispiel 1

1H-NMR ($CDCl_3$): $\delta$ = 2.33 (s, 3H, $CH_3$ aryl); 2.64 (s, 3H, $CH_3$-triazole); 3.88 (d, 1H, J = 5Hz, OH); 4.76, 4.93 (2d, 2H, $J_{AB}$ = 12Hz, $CH_2$-7ring); 5.91 (d, 1H, J = 5Hz, CH); 6.47 (s, 1H, thiophen-H); 7.08-7.45 (m, 8H, aryl-H).

Beispiel 2

1H-NMR ($CDCl_3$): $\delta$ = 2.69 (s, 3H, $CH_3$-triazol); 3.82, 3.88 (2s, 6H, 2-$OCH_3$); 4.97 (s, 2H, $CH_2$-7ring); 6.52-7.47 (m, 7H, aryl-H); 7.20 (s, 1H, thiophen-H).

Beispiel 3

1H-NMR ($CDCl_3$): $\delta$ = 2.76 (s, 3H, $CH_3$-triazol); 3.09 (m, 4H, N-$CH_2$); 3.80 (m, 4H, $OCH_2$); 5.00 (s, 2H, $CH_2$-7ring); 7.10 (s, 1H, thiophen-H); 7.29-7.57 (m, 4H, aryl-H).

Beispiel 4

1H-NMR ($CDCl_3$): $\delta$ = 2.77 (s, 3H, $CH_3$ triazol); 5.01 (s, 2H, $CH_2$-7ring); 7.30-7.58 (m, 4H, aryl-H); 7.32 (s, 1H, thiophen-H); 9.81 (s, 1H, CH = O).

Beispiel 6

1H-NMR ($CDCl_3$): $\delta$ = 2.75 (s, 3H, $CH_3$ triazol); 4.96 (s, 2H, $CH_2$ 7-ring); 6.50-7.58 (m, 11H, aryl-H, thiophen-H, CH = CH).

Beispiel 15

1H-NMR ($CDCl_3$): $\delta$ = 2.63 (s, 3H, $CH_3$ triazol); 4.83 (2d, 2H, $J_{AB}$ = 12Hz, $CH_2$-7-ring); 5.15 (d, 1H, J = 3Hz, OH); 5.91 (d, 1H, J = 3Hz, CH); 6.45 (s, 1H, thiophen-H); 7.15-7.45 (m, 8H, aryl-H).

Beispiel 20

1H-NMR ($CDCl_3$): $\delta$ = 2.67 (s, 3H, $CH_3$-triazol); 3.06 (m, 2H, Ph-$CH_2$); 3.15 (d, 1H, J = 4Hz, OH); 4.88 (2d, 2H, $J_{AB}$ = 12Hz, $CH_2$-7-ring); 5.08 (m, 1H, CH-OH); 6.44 (s, 1H, thiophen-H); 7.10-7.43 (m, 9H, aryl-H).

Beispiel 42

1H-NMR ($CDCl_3$): $\delta$ = 2.50 (s, breit 3H, OH, $NH_2$); 2.69 (s, 3H, $CH_3$ triazol); 2.86, 3.06 (m, 2H, $CH_2$-N); 4.80 (m, 1H, CH-OH); 4.90 (2d, 2H, $J_{AB}$ = 12Hz, $CH_2$-7-ring); 6.50 (s, 1H, thiophen-H); 7.22-7.46 (m, 4H, aryl-H).

Beispiel 47

1H-NMR ($CDCl_3$): δ= 2.31 (s, 3H, N-$CH_3$); 2.54 (m, 4H, $CH_3$-N($CH_2$)$_2$; 2.74 (s, 3H, $CH_3$-triazol); 3.15 (m, 4H, -$SO_2$-N($CH_2$-)$_2$); 4.97 (s, 2H, $CH_2$-7-ring); 7.09 (s, 1H, thiophen-H); 7.26-7.55 (m, 4H, aryl-H).

Beispiel 48

1H-NMR ($CDCl_3$): δ= 2.76 (s, 3H, $CH_3$ triazol); 3.73 (s, 3H, $OCH_3$); 3.89 (s, 2H, $CH_2$-C=O); 4.96 (s, 2H, $CH_2$-7-ring); 7.20 (s, 1H, thiophen-H); 7.31-7.53 (m, 4H, aryl-H); 9.60 (s, breit 1H, NH).

Beispiel 49

1H-NMR (DMSO-d6): δ=2.65 (s, 3H, $CH_3$-triazol); 3.69 (s, 2H, $CH_2$-C=O); 4.90 (s, 2H, $CH_2$-7-ring); 7.12 (s, 1H, thiophen-H); 7.38-7.62 (m, 4H, aryl-H); 8.70 (s, breit, 2H, NH, COOH).

Beispiel 67

1H-NMR ($CDCl_3$): δ = 7.72 - 7.05 (12H, m, aryl-H); 6.55 (1H, s, thiophen-H); 6.71; 6.50 (2H, 2d, J = 11Hz, CH=CH); 4.90 (2H, s, $CH_2$-7-ring); 2.96 (4H, s, $CH_2CH_2$); 2.51 (3H, s, $CH_3$-triazol-ring).

Beispiel 68

1H-NMR($CDCl_3$): δ = 7.47 - 7.14 (4H, m, aryl-H); 6.50 (1H, s, thiophen-H); 5.27 (1H, t, J = 6 HZ, -CH-); 4.94 (2H, s, $CH_2$-7-ring); 4.18(2H, qu, J = 6Hz, $OCH_2$); 2.81 (2H, d, J = 6Hz, $CH_2$-C=0); 2.71 (3H, s, $CH_3$-triazol-ring); 1.69 (1H, s, breit, OH); 1.26 (3H, t, J = 6Hz, $OCH_2$-$CH_3$).

Beispiel 70

1H-NMR ($CDCl_3$): δ 7.51 - 7.14 (14H, m, aryl-H); 7.10 (1H, s, CH=); 6.53 (1H, s, thiophen-H); 4.88 (2H, s, $CH_2$-7-ring); 2.43 (3H, s, $CH_3$-triazol-ring).

Beispiel 71

1H-NMR ($CDCl_3$): δ = 7.43 - 7.19 (4H, m, aryl-H); 6.51 (1H, s, thiophen-H); 4.86 (2H, s, $CH_2$-7-ring); 4.80 (1H, s, breit, OH); 4.76 (2H, s, $CH_2$-O); 2.67 (3H, s, $CH_3$-triazol-ring).

Beispiel 77

1H-NMR ($CDCl_3$) δ = 7.52 - 7.14, 7.01, 6.58 (8H, m, aryl-H); 6.59 (1H, s, thiophen-H); 4.93 (2H, s, $CH_2$-7-ring); 4.44 (2H, d, J = 6Hz, $NCH_2$); 4.17 (1H, t, J = 6Hz, NH); 2,67 (3H, s, $CH_3$-triazol-ring); 2.54 (2H, qu, J = 7Hz, $CH_2CH_3$); 1.18 (3H, t, J = 7Hz, -$CH_2$-$CH_3$).

Beispiel 79

1H-NMR ($CDCl_3$): δ = 7.57 - 7.14; 6.69 - 6.40 (8H, m, aryl-H); 6.61 (1H, s, thiophen-H); 5.87 (1H, tt, JHCF = 54Hz, HCCF = 3Hz)-CH-$F_2$); 4.94 (2H, s, $CH_2$-7-ring); 4.47 (2H, d, J = 6Hz, N-$CH_2$); 4.36 (1H, t, J = 6Hz, NH); 2.67 (3H, s, triazol-ring).

Beispiel 82

1H-NMR ($CDCl_3$): δ = 7.52 - 7.07 (9H, m, aryl-H); 6.40 (1H, s, thiophen-H); 4.90 (2H, s, $CH_2$-7-ring);

4.17 (2H, s, $CH_2$-S); 2,67 (3H, s, $CH_3$-triazol-ring).

Beispiel 84

1H-NMR ($CDCl_3$): $\delta$ = 7.40 - 7.14 (8H, m, aryl-H); 6.36 (1H, s, thiophen-H); 4.90 (2H, s, $CH_2$-7-ring); 4.14 (2H, s, $CH_2$-s); 2.68 (3H, s, $CH_3$-triazol-ring).

Die Verbindungen der allgemeinen Formel I können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowei ein geschmacksverbesserndes Mittel, z.B. Aromastoffe, wie Vanillin oder Orangenextrakt, enthalten.
Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.
Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die nachfolgenden Beispiele illustrieren die vorliegende Erfindung ohne sie jedoch in ihrem Umfang zu beschränken:

Tabletten

1. Die Tablette enthält folgende Bestandteile:

| | |
|---|---|
| Wirkstoff gemäß Formel Ia/Ib | 0,020 Teile |
| Stearinsäure | 0,010 Teile |
| Dextrose | 1,890 Teile |
| gesamt | 1,920 Teile |

Herstellung:

Die Stoffe werden in bekannter Weise zusammengemischt und die Mischung zu Tabletten verpreßt, von denen jede 1,92 g wiegt und 20 mg Wirkstoff enthält.

2. Salbe

Die Salbe setzt sich aus folgenden Bestandteilen zusammen:

| | |
|---|---|
| Wirkstoff gemäß Formel Ia/Ib | 50 mg |
| Neribas Salbe (Handelsware Scherax) | ad 10 g |

Herstellung:

Der Wirkstoff wird mit 0,5 g Salbengrundlage verrieben und die restliche Grundlage in Teilmengen zu 1,0 g nach und nach innig zu einer Salbe vermischt. Man erhält eine 0,5 %ige Salbe. Die Verteilung des Wirkstoffes in der Grundlage wird optisch unter dem Mikroskop kontrolliert.

3. Creme

| Zusammensetzung: | |
|---|---|
| Wirkstoff gemäß Formel Ia/Ib | 50 mg |
| Neribas Salbe (Handelsware Scherax) | ad 10 g |

Herstellung

Der Wirkstoff wird mit 0,5 g Cremegrundlage verrieben und die restliche Grundlage in Teilmengen zu 1,0 g nach und nach mit Pistill eingearbeitet. Man erhält eine 0,5%ige Creme. Die Verteilung des Wirkstoffes in der Grundlage wird optisch unter dem Mikroskop kontrolliert.

4. Ampullenlösung

| Zusammensetzung: | |
|---|---|
| Wirkstoff gemäß Formel Ia/Ib | 1,0 mg |
| Natriumchlorid | 45,0 mg |
| Aqua pro inj. ad | 5,0 ml |

Herstellung:

Der Wirkstoff wird bei Eigen-pH oder gegebenenfalls bei pH 5,5 bis 6,5 in Wasser gelöst und Natriumchlorid als Isotonanz zugegeben. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 1 mg, 5 mg und 10 mg Wirkstoff.

5. Suppositorien

Jedes Zäpfchen enthält:

| | |
|---|---|
| Wirkstoff gemäß Formel Ia/Ib | 1,0 Teile |
| Kakaobutter (Fp.36-37° C) | 1200,0 Teile |
| Carnaubawachs | 5,0 Teile |

Herstellung

Kakaobutter und Carnaubawachs werden zusammengeschmolzen. Bei 45° C gibt man den Wirkstoff hinzu und rührt, bis eine komplette Dispersion entstanden ist.
Die Mischung wird in Formen entsprechender Größe gegossen und die Zäpfchen zweckmäßig verpackt.

6. Inhalationslösungen

| Zusammensetzung: | |
|---|---|
| a) Wirkstoff gemäß Formel Ia/Ib | 500 mg |
| Na-EDTA | 50 mg |
| Benzalkoniumchlorid | 25 mg |
| Natriumchlorid | 880 mg |
| destilliertes Wasser ad | 100 ml |

Herstellung:

96 % der Wassermenge werden vorgelegt, darin nacheinander Na-EDTA, Benzalkoniumchlorid, Natriumchlorid und Wirkstoff klar gelöst und mit dem restlichen Wasser aufgefüllt. Die Lösung wird in 20 ml-Tropfflaschen abgefüllt. Eine Dosis (20 Tropfen, 1 ml) enthält 5 mg Wirkstoff.

| | |
|---|---|
| b) Wirkstoff gemäß Formel Ia/Ib | 500 mg |
| Natriumchlorid | 820 mg |
| destilliertes Wasser ad | 100 ml |

Herstellung

96 % der Wassermenge werden vorgelegt, darin nacheinander der Wirkstoff und Natriumchlorid gelöst, mit dem restlichen Wasser aufgefüllt und die Lösung in Eindosenbehälter (4 ml) abgefüllt. Die Lösung enthält 20 mg Wirkstoff.

## Ansprüche

1) Neue Thienodiazepine der allgemeinen Formel

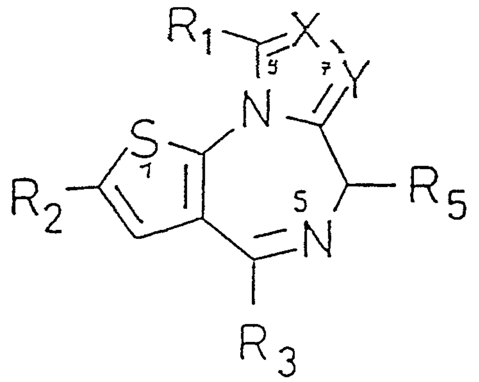

Ia

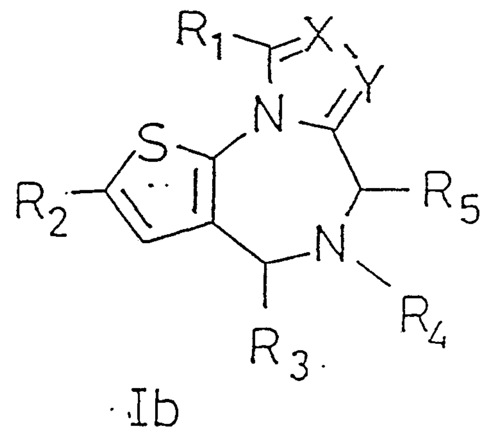

Ib

worin

$R_1$ Wasserstoff, eine verzweigte oder unverzweigte Alkylgruppe mit 1 bis 4 Kohlenstoffatomen, bevorzugt Methyl, die gegebenenfalls durch Hydroxy oder Halogen substituiert sein kann, eine Cyclopropylgruppe, eine Cyclobutyl, eine verzweigte oder unverzweigte Alkoxygruppe mit 1 bis 4 Kohlenstoffatomen, bevorzugt Methoxy, Halogen, bevorzugt Chlor oder Brom;

$R_2$ $$Ra - A - \underset{\displaystyle R_b}{\overset{\displaystyle OH}{\overset{|}{\underset{|}{C}}}} -$$ oder

$$Ra - B - \underset{R_c}{C} = \underset{R_b}{C} -$$ oder

$$Rd - N = \underset{H}{C} -$$

oder

$Re - D - CH_2-$

worin

A $-(CH_2)_n-$ oder eine verzweigte Alkylgruppe mit n Kohlenstoffatomen

B $-(CH_2)_m-$ oder eine verzweigte Alkylgruppe mit m Kohlenstoffatomen

D Sauerstoff, Schwefel oder NH bedeutet,

Ra Wasserstoff, verzweigtes oder unverzweigtes Alkyl mit 1 bis 4 Kohlenstoffatomen, gegebenenfalls durch Halogen substituiert, $C_1$ bis $C_8$ Alkoxy, bevorzugt $C_1$ bis $C_4$-Alkoxy, eine gegebenenfalls substituierte Cycloalkylgruppe mit 3 bis 6 Kohlenstoffatomen, gegebenenfalls substituiertes Aryl-, Aryloxy-, Aralkyl-, Aralkyloxy, gegebenenfalls substituiertes Heteroaryl, ein gegebenenfalls substituierter gesättigter oder ungesättigter 5, 6- oder 7- gliedriger heterocyclischer Ring, einen Rest der Formel

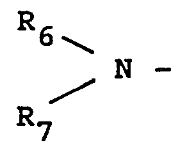

wobei

$R_6$ Wasserstoff, eine verzweigte oder unverzweigte Alkyl-, Alkenyl oder Alkinylgruppe mit 1 - 10 Kohlenstoffatomen, bevorzugt 1 - 4, Kohlenstoffatomen, die gegebenenfalls durch Halogen, Carboxy, Alkoxycarbonyl mit 1 bis 6, bevorzugt 1 bis 4, Kohlenstoffatomen, Phenyl, substituiertes Phenyl, oder durch einen C- oder N-verknüpften Heterocyclus substituiert sein kann, wobei die Kohlenstoffkette durch Stickstoff, Sauerstoff oder Schwefel unterbrochen sein kann,

eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,

$R_6$ eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen,

$R_6$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6-, oder 7-gliedriger heterocyclischer Ring, Phenyl, substituiertes Phenyl;

$R_7$ Wasserstoff, eine verzweigte oder unverzweigte Alkyl-, Alkenyl oder Alkinylgruppe mit 1 - 10 Kohlenstoffatomen, bevorzugt 1 -4 Kohlenstoffatomen, die gegebenenfalls durch Halogen, Carboxy, Alkoxycarbonyl, Phenyl, substituiertes Phenyl, oder durch einen C- oder N-verknüpften Heterocyclus substituiert sein kann, wobei die Kohlenstoffkette durch Stickstoff, Sauerstoff oder Schwefel unterbrochen sein kann,

eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,

$R_7$ eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen, oder

$R_6$ und $R_7$ bilden zusammen mit dem Stickstoffatom einen gesättigten oder ungesättigten gegebenenfalls ein- oder mehrfach

durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;

$R_7$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6-, oder 7-gliedriger heterocyclischer Ring, Phenyl, substituiertes Phenyl;

$R_a$ ein gegebenenfalls substituierter Imido- oder Phthalimidorest;

$R_b$ entsprechend einem Rest wie unter $R_a$ definiert, bevorzugt Wasserstoff oder verzweigtes oder unverzweigtes Alkyl mit 1 bis 4 Kohlenstoffatomen, Phenyl, substituiertes Phenyl;

$R_c$ Wasserstoff, Aryl, bevorzugt Phenyl, substituiertes Phenyl, $C_1$ - $C_4$-Alkyl,

$R_d$ eine verzweigte oder unverzweigte Alkyl-, Alkenyl- oder Alkinylgruppe mit 1 bis 10 Kohlenstoffatomen, bevorzugt mit 5 bis 10 Kohlenstoffatomen, im Substitutionsfall bevorzugt mit 1 bis 4 Kohlenstoffatomen, die gegebenenfalls durch Carboxy, Alkoxycarbonyl mit 1 bis 6 , bevorzugt 1 bis 4 Kohlenstoffatomen, Phenyl, substituiertes Phenyl, oder durch einen C- oder N-verknüpften 5-, 6-, 7-gliedrigen Heterocyclus substituiert sein kann,

$R_d$ ein gegebenenfalls ein- oder mehrfach durch verzweigtes oder unverzweigtes Alkyl mit 1 bis 4 Kohlenstoffatomen substituierter gesättigter oder ungesättigter über ein Kohlenstoffatom oder Stickstoffatom verknüpfter 5-, 6- oder 7-gliedriger heterocyclischer Ring, Phenyl, substituiertes Phenyl;

$R_e$ für den Fall, daß D die Bedeutung NH aufweist bedeutet, $R_e$ = $R_d$,

für den Fall, daß D die Bedeutung Sauerstoff oder Schwefel aufweist, bedeutet,

$R_e$ $C_1$ - $C_8$, bevorzugt $C_1$ - $C_4$ - Alkyl, gegebenenfalls substituiert durch Phenyl oder substituiertes Phenyl;

Phenyl, substituiertes Phenyl
n eine der Zahlen 0, 1, 2, 3, 4, 5, 6, 7 oder 8, bevorzugt 0, 1 oder 2;
m eine der Zahlen 0, 1, 2, 3, 4, 5, 6 oder 7 bevorzugt 0, 1 oder 2;
$R_2$ ein Rest der allgemeinen Formel
$R_f$-O-$SO_2$-
worin
$R_f$ verzweigtes oder unverzweigtes Alkyl mit 1 bis 10, bevorzugt 1 bis 4 Kohlenstoffatomen, das gegebenenfalls durch Halogen, Phenyl, substituiertes Phenyl substituiert sein kann;
eine Cycloalkylgruppe mit 1 bis 6 Kohlenstoffatomen, gegebenenfalls substituiert durch Halogen, Phenyl oder substituiertes Phenyl,
$R_f$ Phenyl, substituiertes Phenyl, Heteroaryl, substituiertes Heteroaryl;
$R_2$ einen Rest der allgemeinen Formel

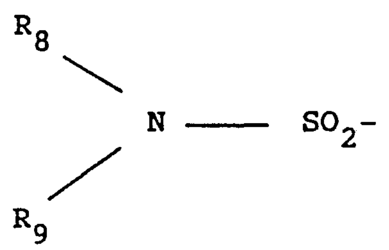

worin
$R_8$ Wasserstoff, eine verzweigte oder unverzweigte gegebenenfalls durch Stickstoff, Sauerstoff oder Schwefel unterbrochene Alkyl-, Alkenyl oder Alkinylgruppe mit 1 - 10 Kohlenstoffatomen, bevorzugt 1 -4 Kohlenstoffatomen, die gegebenenfalls durch {Halogen, Hydroxy, Phenyl, substituiertes Phenyl, Alkyloxycarbonyl mit 1 bis 8 - bevorzugt 1-4 -Kohlenstoffatomen in der Alkylkette, Carboxy, eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,
eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen, oder durch einen C- oder N-verknüpften Heterocyclus} substituiert sein kann;
$R_8$ Phenyl, substituiertes Phenyl
$R_8$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6-, oder 7-gliedriger heterocyclischer Ring;
$R_9$ Wasserstoff, eine verzweigte oder unverzweigte gegebenenfalls durch Stickstoff, Sauerstoff oder Schwefel unterbrochene Alkyl-, Alkenyl oder Alkinylgruppe mit 1 - 10 Kohlenstoffatomen, bevorzugt 1 -4 Kohlenstoffatomen, die gegebenenfalls durch {Halogen, Hydroxy, Carboxy, Alkoxycarbonyl, Phenyl, substituiertes Phenyl, eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy} substituiert sein kann,
eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen, oder durch einen C- oder N-verknüpften Heterocyclus substituiert sein kann,
$R_9$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoffatom verknüpfter 5-, 6-, oder 7-gliedriger heterocyclischer Ring, Phenyl, substituiertes Phenyl; oder
$R_8$ und $R_9$ bilden zusammen mit dem Stickstoffatom einen gesättigten oder ungesättigten gegebenenfalls ein- oder mehrfach
durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;
$R_a$ einen Rest der allgemeinen Formel

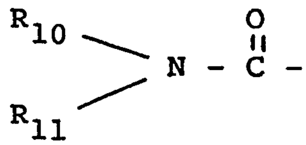

worin

$R_{10}$ und $R_{11}$, die gleich oder verschieden sein können, Wasserstoff, Phenyl, substituiertes Phenyl, eine gegebenenfalls substituierte Cycloalkylgruppe mit 3 bis 6 Kohlenstoffatomen, eine verzweigte oder unverzweigte Alkyl-, Alkenyl-oder Alkinylgruppe mit 1 bis 10 - bevorzugt 1 - 4 - Kohlenstoffatomen, die gegebenenfalls durch {Halogen, Hydroxy, Nitro, Phenyl, substituiertes Phenyl, Amino, substituiertes Amino, Alkoxy 1 - 8, bevorzugt 1 - 4 oder im Fall von $R_{10}$ = Wasserstoff und Alkyl durch eine Esterfunktion oder ein Säureamid der allgemeinen Formel

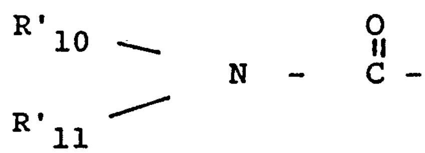

worin

$R'_{10}$ und $R'_{11}$ dieselbe Bedeutung wie $R_{10}$ und $R_{11}$, haben kann, Phenyl, substituiertes Phenyl} substituiert sein kann;

$R_{10}$ oder $R_{11}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoff gebundener 5-, 6- oder 7-gliedriger heterocyclischer Ring;

oder

$R_{10}$ und $R_{11}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann.

$R_2$ -CHO oder COOH;

$R_3$ Phenyl, wobei der Phenylring ein oder mehrfach, bevorzugt in 2-Stellung, durch Methyl, bevorzugt Halogen, bevorzugt Brom, besonders bevorzugt Chlor, Nitro und/oder Trifluormethyl substituiert sein kann;

$R_3$ einen Rest der allgemeinen Formel

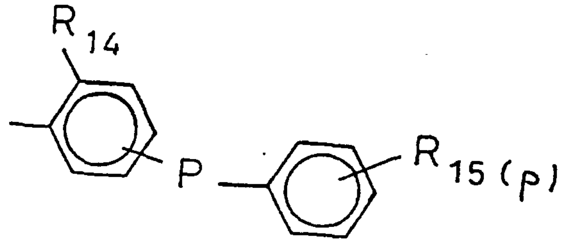

worin

$R_{14}$ Wasserstoff oder Chlor

P den Rest - $(CH_2)_m$- mit m = 0, 1, 2, 3 oder 4, $-OCH_2-$, $-CH_2OCH_2-$

P 0, 1, 2, oder 3

$R_{15}$ Wasserstoff, $CF_3$, Chlor, Fluor, $C_1$ - $C_4$-Alkyl, $C_1$ - $C_4$ - Alkoxy, oder den Rest

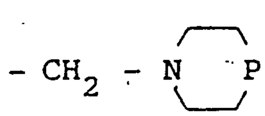

worin P, Sauerstoff, Schwefel oder $N\text{-}CH_3$ bedeutet,
mit der Maßgabe, daß wenn $R_{14}$ Wasserstoff bedeutet, $R_{15}$, in der 3 oder 4 Position ist, wenn $R_{14}$ Chlor bedeutet, $R_{15}$ in der 4 Position ist, wenn $R_{15}$ Chlor, Fluor, oder Methyl bedeutet, ist p 3, wenn $R_{15}$ tert.-Butyl oder

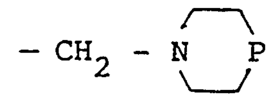

bedeutet ist p = 1; wenn p = 2 ist, ist $R_{15}$ nicht gleichzeitig in der 2- oder 6-Position;
$R_3$ Pyridyl, substituiertes Pyridyl, Thienyl oder substituiertes Thienyl, wobei der Heterocyclus bevorzugt über die 2-Stellung verknüpft ist;
$R_4$ Wasserstoff, verzweigtes oder unverzweigtes Alkyl mit 1 bis 4 Kohlenstoffatomen, Formyl, verzweigter oder unverzweigter Acylrest mit 1 bis 4 Kohlenstoffatomen;
$R_5$ Wasserstoff, Hydroxy, verzweigtes oder unverzweigtes Alkyl mit 1 bis 6, bevorzugt 1 bis 4 Kohlenstoffatomen, besonders bevorzugt Methyl, gegebenenfalls ist die Alkylgruppe durch Hydroxy, Alkylsulfonyloxy, bevorzugt Methylsulfonyloxy, $-NR_8R_9$ oder $-CO\text{-}NR_{12}R_{13}$ substituiert,
wobei $R_8$ und $R_9$ die zuvor genannte Bedeutung aufweisen und
$R_{12}$ Wasserstoff, eine verzweigte oder unverzweigte Alkyl-, Alkenyl- oder Alkinylgruppe mit 1 bis 18 Kohlenstoffatomen, bevorzugt mit 1 bis 6, besonders bevorzugt 1 bis 4, die gegebenenfalls durch Halogen, Hydroxy, Nitro, Amino, substituiertes Amino, Alkoxy, bevorzugt Methoxy oder im Fall von $R_{13}$ = Wasserstoff oder Alkyl, durch eine Esterfunktion oder durch ein Säureamid der allgemeinen Formel

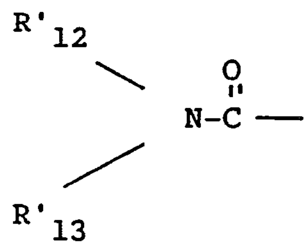

worin $R'_{12}$ und $R'_{13}$, dieselbe Bedeutung wie $R_{12}$ und $R_{13}$ haben können, Phenyl, substituiertes Phenyl, substituiert sein kann;
$R_{13}$ Wasserstoff, eine verzweigte oder unverzweigte Alkyl-, Alkenyl- oder Alkinylgruppe mit 1 bis 18 Kohlenstoffatomen, bevorzugt mit 1 bis 6, besonders bevorzugt 1 bis 4, die gegebenenfalls durch Halogen, Hydroxy, Nitro, Amino, substituiertes Amino, Alkoxy, bevorzugt Methoxy; Phenyl substituiert sein kann;
ein gegebenenfalls ein- oder mehrfach durch verzweigtes oder unverzweigtes Alkyl mit 1 bis 4 Kohlenstöffatomen substituierter, gesättigter oder ungesättigter über ein Kohlenstoffatom oder Stickstoffatom verknüpfter 5-, 6-, oder 7-gliedriger heterocyclischer Ring;
oder
$R_{12}$ und $R_{13}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann,
$R_5$ Alkylcarbonyloxy mit 1 bis 4 Kohlenstoffatomen in der verzweigten oder unverzweigten Alkylkette, Alkoxycarbonyl mit 1 bis 4 Kohlenstoffatomen in der verzweigten oder unverzweigten Alkylkette, Carboxyalkyl mit 1 bis 4 Kohlenstoffatomen in der verzweigten oder unverzweigten Alkylkette, Alkoxycarbonylalkyl mit 1 bis 4 Kohlenstoffatomen in den verzweigten oder unverzweigten Alkylresten;
X/Y unabhängig voneinander $C\text{-}R_1$ oder N, aber nicht beide $C\text{-}R_1$, mit $R_1$ bevorzugt Wasserstoff oder Methyl,
oder Y die Gruppe $C\text{-}COOR'$, wobei $R'$ Alkyl oder Wasserstoff bedeutet und X Stickstoff bedeuten können;
gegebenenfalls in Form ihrer Racemate, ihrer Enantiomere, ihrer Diastereomere und ihrer Gemische sowie gegebenenfalls ihre physiologisch unbedenklichen Säureadditionssalze.

2) Neue Thienodiazepine der allgemeinen Formel Ia gemäß Anspruch 1
worin

$R_1$ eine Methylgruppe, eine Ethylgruppe die gegebenenfalls durch Hydroxy oder Halogen substituiert sein können, eine Cyclopropylgruppe, eine Methoxygruppe;

$R_2$ 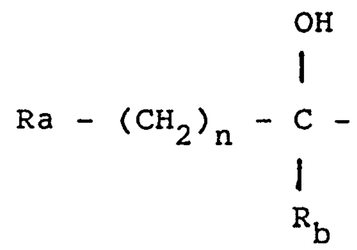 oder

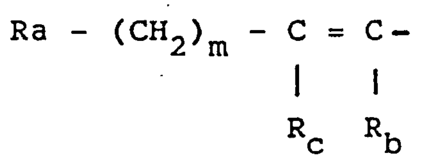 oder

Re - D - $CH_2$ -
worin D wie zuvor definiert ist,
oder
$R_d$ - N = CH -
Ra Wasserstoff, verzweigtes oder unverzweigtes Alkyl mit 1 bis 4 Kohlenstoffatomen gegebenenfalls durch Halogen substituiert, Phenyl, gegebenenfalls ein-oder mehrfach substituiert durch Halogen, Amino, Alkylamino, Dialkylamino, $C_1$-$C_4$ Alkyl $C_1$-$C_4$-Halogenalkyl, $C_1$-$C_4$ Alkoxy und/oder $C_1$-$C_4$-Alkoxycarbonyl, gegebenenfalls substituiertes Benzodioxolan, gegebenenfalls substituiertes Furan, Thiophen oder Pyridin, einen Rest der Formel

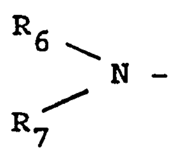

wobei
$R_6$ Wasserstoff, eine verzweigte oder unverzweigte Alkylgruppe mit 1 -4 Kohlenstoffatomen, die gegebenenfalls durch Halogen, Phenyl, substituiertes Phenyl, substituiert sein kann, eine Allylgruppe, eine Propargylgruppe eine verzweigte oder unverzweigte Alkylcarbonylgruppe mit 1-4 Kohlenstoffatomen, wobei die Alkylgruppe durch Halogen oder Hydroxy substituiert sein kann,
$R_6$ eine gegebenenfalls substituierte Phenylcarbonyl, eine Phenylsulfonyl oder Tolylsulfonyl, eine Alkylsulfonylgruppe mit 1 bis 4 Kohlenstoffatomen,
$R_7$ Wasserstoff, eine verzweigte oder unverzweigte Alkyl- und Alkenyl mit 1 - 4 Kohlenstoffatomen;
oder
$R_6$ und $R_7$ bilden zusammen mit dem Stickstoffatom einen gesättigten gegebenenfalls ein- oder zweifach durch verzweigte oder unverzweigte Alkylgruppen mit 1 bis 3 Kohlenstoffatomen substituierten 5-, oder 6-Ring der als weitere Heteroatome Stickstoff oder Sauerstoff enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;
$R_a$ ein Phthalimidorest;
$R_b$ Wasserstoff oder verzweigtes oder unverzweigtes Alkyl mit 1 bis 4 Kohlenstoffatomen, Phenyl, gegebenenfalls substituiert durch Halogen, $C_1$-$C_4$-Alkyl $C_1$-$C_4$-Halogen- alkyl, $C_1$-$C_4$-Alkoxy, $C_1$-$C_4$ Alkoxycarbonyl, Amino, Alkylamino, Dialkylamino;
$R_c$ Wasserstoff oder Phenyl
$R_e$/$R_d$
Phenyl gegebenenfalls substituiert durch $C_1$ - $C_4$-Alkyl, $C_1$ - $C_4$-Halogenalkyl, $C_1$ - $C_4$-Alkoxy oder $C_1$ -$C_4$-

Halogenalkoxy,
n eine der Zahlen 0, 1, 2, 3, 4, 5, 6, 7 oder 8, bevorzugt 0, 1 oder 2;
m eine der Zahlen 0, 1, 2, 6 oder 7;
$R_2$ ein Rest der allgemeinen Formel
$R_f$-O-$SO_2$-
worin
$R_f$ verzweigtes oder unverzweigtes Alkyl mit 1 bis 4 Kohlenstoffatomen, das gegebenenfalls durch Halogen, gegebenenfalls ein- oder mehrfach durch Halogen, Amino, Alkylamino, Dialkylamino, $C_1$-$C_4$ Alkyl $C_1$-$C_4$-Halogenalkyl, $C_1$-$C_4$ Alkoxy und/oder $C_1$-$C_4$ -Alkoxycarbonyl substituiertes Phenyl substituiert sein kann;
$R_f$ Phenyl, substituiertes Phenyl, Heteroaryl, substituiertes Heteroaryl;
$R_2$ einen Rest der allgemeinen Formel

$$R_f\text{-O-}SO_2\text{-}$$

worin
$R_8$ Wasserstoff, eine verzweigte oder unverzweigte Alkylgruppe mit 1 -4 Kohlenstoffatomen, die gegebenenfalls durch Halogen, Hydroxy, Phenyl, substituiertes Phenyl, eine verzweigte oder unverzweigte Alkylcarbonylgruppe mit 1-4 Kohlenstoffatomen, ein gegebenenfalls substituiertes Phenylcarbonyl, gegebenenfalls substituiertes Phenylsulfonyl oder Tolylsulfonyl, eine Alkylsulfonylgruppe mit 1 bis 4 Kohlenstoffatomen, substituiert sein kann.
$R_9$ Wasserstoff, oder
$R_8$ und $R_9$ bilden zusammen mit dem Stickstoffatom einen gesättigten gegebenenfalls ein- oder zweifach durch verzweigte oder unverzweigte Alkylgruppen mit 1 bis 4 Kohlenstoffatomen substituierten 5-oder 6-Ring, der als weiteres Heteroatom Stickstoff oder Sauerstoff enthalten kann, wobei jedes weitere Stickstoffatom gegebenenfalls durch Methyl, Ethyl oder Isopropyl substituiert sein kann;
$R_2$ -CHO oder COOH;
$R_3$ Phenyl, wobei der Phenylring in 2-Stellung durch Halogen, bevorzugt Brom, besonders bevorzugt Chlor substituiert sein kann;
$R_3$ den 4-[2-(3,4,5-Trimethoxyphenyl)-ethyl]-phenyl-Rest
$R_5$ Wasserstoff, Hydroxy, oder Methyl,
X/Y beide Stickstoff oder X = CH und Y = Stickstoff.

3) Verfahren zur Herstellung von Verbindungen der allgemeinen Formel Ia und Ib wie in Anspruch 1 oder 2 definiert, dadurch gekennzeichnet, daß man eine Verbindung der allgemeinen Formel II

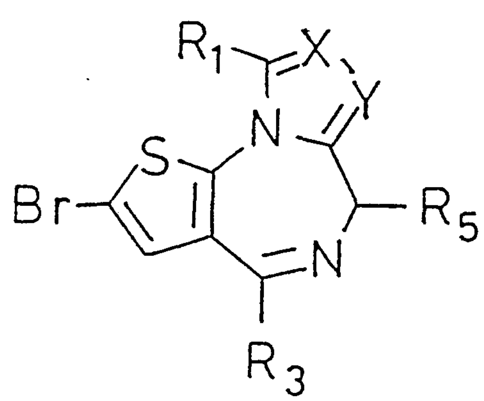

worin
$R_1$ Wasserstoff, eine verzweigte oder unverzweigte Alkylgruppe mit 1 bis 4 Kohlenstoffatomen, bevorzugt Methyl, eine Cyclopropylgruppe, eine Cyclobutyl, eine verzweigte oder unverzweigte Alkoxygruppe mit 1 bis 4 Kohlenstoffatomen, bevorzugt Methoxy und $R_3$ und $R_5$ wie in Anspruch 1 definiert sind mit zwei Äquivalenten einer metallorganischen Verbindung, insbesondere eines Grignardreagenzes - bevorzugt

ausgewählt aus der Gruppe, Ethylmagnesiumbromid, Ethylmagnesiumjodid, Methylmagnesiumbromid, Methylmagnesiumjodid und Phenylmagnesiumbromid - umsetzt, anschließend das so erhaltene Produkt mit
a) Aldehyden oder Ketonen umsetzt und gegebenenfalls die so erhaltenen Carbinole dehydratisiert oder
b) mit Schwefeldioxid und anschließend mit Chlor umsetzt, das so erhaltene Sulfonsäurechlorid mit
b1) Aminen zu Sulfonamiden oder
b2) Alkoholen zu Sulfonestern umsetzt
oder
c) mit Kohlensäure zur Carbonsäure
oder
d) mit Dimethylformamid zum Aldehyd
e) im Sinne einer palladiumkatalysierten Wurtz-Reaktion zu Verbindungen mit $R_2$ = Ra-B-CRc = Crb- umsetzt und die so erhaltenen Derivate gegebenenfalls nach an sich bekannten Verfahren in Endprodukte der allgemeinen Formel I ihre optisch aktiven Isomere wie auch ihre Säureadditionssalze überführt.

4) Metallierte Verbindung hergestellt durch Umsetzung von zwei Äquivalenten eines metallorganischen Reagenzes, bevorzugt eines Grignardreagenzes der Formel
$A^x$Mg Hal
worin $A^x$ ein geeigneter organischer Rest und Hal ein Halogenid - bevorzugt Brom oder Jod ist, mit einem Äquivalent eines Thienodiazepins der allgemeinen Formel

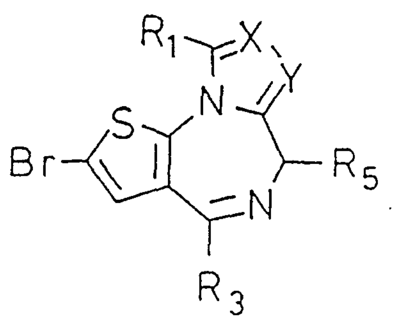

II

worin
$R_1$ Wasserstoff, eine verzweigte oder unverzweigte Alkylgruppe mit 1 bis 4 Kohlenstoffatomen, bevorzugt Methyl, eine Cyclopropylgruppe, eine Cyclobutyl, eine verzweigte oder unverzweigte Alkoxygruppe mit 1 bis 4 Kohlenstoffatomen, bevorzugt Methoxy, und $R_3$ und $R_5$ wie in Anspruch 1 definiert sind.

5) Pharmazeutische Präparate, enthaltend als Wirkstoffe Verbindungen der allgemeinen Formel Ia oder Ib in Kombination mit üblichen Hilfs- und/oder Trägerstoffen.

6) Verfahren zur Herstellung von pharmazeutischen Präparaten nach Anspruch 5, dadurch gekennzeichnet, daß man Verbindungen der allgemeinen Formel Ia oder Ib mit üblichen galenischen Hilfs- und/oder Trägerstoffen zu üblichen pharmazeutischen Anwendungsformen verarbeitet.

7) Verbindungen der allgemeinen Formel Ia oder Ib zur Verwendung für die Herstellung von Arzneimitteln mit PAF-antagonistischer Wirkung.

8) Methode zur Behandlung pathologischer Zustände und Krankheiten, an denen PAF (Plättchen Aktivierender Faktor) beteiligt ist, dadurch gekennzeichnet, daß man eine Verbindung der allgemeinen Formel Ia und Ib verwendet.

9) Verfahren zur Herstellung von Arzneimitteln, enthaltend Verbindungen der allgemeinen Formel Ia oder Ib zur Behandlung von Krankheiten an denen PAF beteiligt ist.

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung
EP 90103948.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| X | PATENT ABSTRACTS OF JAPAN Band 1, Nr. 111 (C-77), 26. September 77; & JP - A - 52 71494 (YOSHITOMI SEIYAKU K. K.) 14.06.1977 --- | 1 | C07D495/14<br>A61K31/55<br>//(C07D495/14,<br>333:00,249:00,<br>243:00) |
| X | DE - A - 2503235 (C.H. BOEHRINGER SOHN) * Seiten 5,6, Beispiele 1,6 * --- | 1 | |
| X | WO - A - 8809333 (YOSHITOMI PHARMACEUTICAL INDUSTRIES LTD) * Zusammenfassung * --- | 1 | |
| A,D | EP - A - 0230942 (BOEHRINGER INGELHEIM KG) * Ansprüche 1,2,8-10,12 * --- | 1,2,5-7, 9 | |
| A | EP - A - 0268242 (YOSHITOMI PHARMACEUTICAL INDUSTRIES LTD) * Ansprüche 1,4-7; Seiten 7-14 * --- | 1,5-7,9 | |
| A | EP - A - 0194416 (BOEHRINGER INGELHEIM KG) * Ansprüche 1,7-9,11 * --- | 1,5-7,9 | RECHERCHIERTE SACHGEBIETE (Int. Cl.⁵)<br>C07D495/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7,9
Unvollständig recherchierte Patentansprüche: ---
Nicht recherchierte Patentansprüche: 8
Grund für die Beschränkung der Recherche:

Methode zur chirugischen oder therapeutischen Behandlung des menschlichen oder tierischen Körpers (Art. 42(4) EPC).

| Recherchenort | Abschlußdatum der Recherche | Prüfer |
|---|---|---|
| Berlin | 07.06.1990 | C.V.F. HASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument